# EUROPEAN PATENT APPLICATION

(11) **EP 3 279 664 A1**
(43) Date of publication of application: **07.02.2018**
(21) Application number: 16183098.9
(22) Date of filing: 05.08.2016
(51) Int. Cl.: G01N 33/569

(54) **MEANS AND METHODS FOR DETECTING BETA-1,6-GLUCAN**

(71) Applicant: Universität zu Köln, 50923 Köln (DE)
(72) Inventor: Zuccaro, Alga, 50968 Köln (DE); Wawra, Stephan, 50937 Köln (DE)
(74) Representative: Herzog, Fiesser & Partner Patentanwälte PartG mbB

(57) **Abstract**

The present invention relates generally to the field of molecular biology and concerns a conjugate comprising a beta 1,6-glucan binding polypeptide, or a beta 1,6-glucan binding fragment thereof, and a detectable label. Moreover, also encompassed by the invention are a fragment of the beta 1,6-glucan binding polypeptide that specifically binds to beta 1,6 glucans, a vector comprising a nucleic acid encoding for the beta 1,6-glucan binding polypeptide and a host cell or non-human organism comprising a nucleic acid encoding for the beta 1,6-glucan binding polypeptide and the use of said conjugate comprising a beta 1,6-glucan binding polypeptide, or a beta 1,6-glucan binding fragment thereof.

## Description

### FIELD OF THE INVENTION

The present invention relates generally to the field of molecular biology and concerns a conjugate comprising a beta 1,6-glucan binding polypeptide, or a beta 1,6-glucan binding fragment thereof, and a detectable label. Moreover, also encompassed by the invention are a fragment of the beta 1,6-glucan binding polypeptide that specifically binds to beta 1,6 glucans, a vector comprising a nucleic acid encoding for the beta 1,6-glucan binding polypeptide and a host cell or non-human organism comprising a nucleic acid encoding for the beta 1,6-glucan binding polypeptide and the use of said conjugate comprising a beta 1,6-glucan binding polypeptide, or a beta 1,6-glucan binding fragment thereof.

### BACKGROUND OF THE INVENTION

Glucans are polysaccharides in particular consisting of D-glucose units. In general, in these glucans the D-glucose rings are connected with each other at varying carbon positions depending on the source. The glucans may have a linear or branched backbone.

Depending on their composition, β-glucans can vary with respect to their molecular mass, solubility, viscosity, branching structure, and gelation properties. Beta-glucans have been shown to occur in the cell walls of cereals, yeast, bacteria, and fungi and are well-known modulators of the immune system in mammals.

Typically, such β-glucans form a linear backbone with 1,3 β-glycosidic bonds. However, some beta-glucans, such as e.g. yeast and fungal β-glucans, also contain 1-6 bonds, such as e.g. 1,6 side branches (1,6 β-glycosidic bonds). β-Glucans of yeast typically contain a 1,3 carbon backbone with elongated 1,6 carbon branches (Manners, David J. (2 February 1973). "The Structure of a β-(1->3)-D-Glucan from Yeast Cell Walls". Biochemistry.)

A series of human clinical trials in the 1990s evaluated the impact of PGG-glucan (Poly-[1-6]--D-glucopyranosyl-[1-3]--D-glucopyranose), isolated from the cell wall of Saccharomyces cerevisiae, on infections in high-risk surgical patients. For example, PGG glucan, was shown to exert broad-spectrum anti-infective activities without cytokine induction and reduce complications (Patchen et al., Exp Hematol. 1998 Dec;26(13):1247-54; Kernodle et al. Antimicrob. Agents Chemother. March 1998 vol. 42 no. 3 545-549).

Diagnostic tests based on 1,3-beta-D-glucan or Chitin for invasive fungal infections and pneumonia caused by Pneumocystis jiroveci (carinii) are available on the market, i.e. the Fungitell® ß-D-Glucan Assay (Fungitell is a product and registered trademark of Associates of Cape Cod, Inc.). However, agents specifically binding to beta 1,6-glucans that could be used in a method for diagnosing fungal infection in a subject are currently not known.

Most studies on fungal beta-glucans as modulators of the immune system have been performed in mammals. However, little is known about beta-glucan triggered immunity in plants.

To protect and defend against microbial invaders, but also to start the symbiotic program, plants need to detect the presence of microbes by means of pattern recognition receptors (PRR's) that respond to specific microbe-associated molecular patterns (MAMPs) (Jones JD, Dangl JL. The plant immune system. Nature 2006, 444(7117): 323-329; Limpens E, van Zeijl A, Geurts R. Lipochitooligosaccharides Modulate Plant Host Immunity to Enable Endosymbioses. Annu Rev Phytopathol 2015, 53: 311-334). MAMPs are usually slowly evolving components of microbes with crucial biological functions that are not present in the host (Nurnberger T, Brunner F, Kemmerling B, Piater L. Innate immunity in plants and animals: striking similarities and obvious differences. Immunological reviews 2004, 198: 249-266). During plant colonization the first microbial derived structure that makes physical contact with the host cell is the cell wall. In pathogenic as well as in beneficial fungi an important structural building block of the cell wall is chitin, a well-known elicitor of immune responses in plants (Sanchez-Vallet A, Mesters JR, Thomma BP. The battle for chitin recognition in plant-microbe interactions. FEMS microbiology reviews 2015, 39(2): 171-183). Over the past 10 years, several plant receptors for chitin have been identified and characterized as have different microbial strategies to evade chitin recognition, including modification of the cell wall carbohydrate composition and secretion of effectors (van den Burg HA, Harrison SJ, Joosten MH, Vervoort J, de Wit PJ. Cladosporium fulvum Avr4 protects fungal cell walls against hydrolysis by plant chitinases accumulating during infection. Mol Plant Microbe Interact 2006, 19(12): 1420-1430; de Jonge R, van Esse HP, Kombrink A, Shinya T, Desaki Y, Bours R, et al. Conserved fungal LysM effector Ecp6 prevents chitin-triggered immunity in plants. Science 2010, 329(5994): 953-955; Sanchez-Vallet A, Saleem-Batcha R, Kombrink A, Hansen G, Valkenburg DJ, Thomma BP, et al. Fungal effector Ecp6 outcompetes host immune receptor for chitin binding through intrachain LysM dimerization. eLife 2013, 2: e00790). In spite of its strong elicitor activity, chitin represents only a small percentage of the fungal cell wall. By far the most abundant cell wall polysaccharide in the majority of fungi is β-glucan, a well characterized elicitor in fungus-animal systems (Brown GD, Gordon S. Fungal beta-glucans and mammalian immunity. Immunity 2003, 19(3): 311-315). Most β-glucans originating from the fungal cell wall are made of glucosyl units joined by β-1,3-linkages often branched via β-1,6-linked glucose residues (Fesel PH, Zuccaro A. beta-glucan: Crucial component of the fungal cell wall and elusive MAMP in plants. Fungal genetics and biology : FG & B 2015). Whereas β-1,3-glucan is found also in the cell wall of plants, β-1,6-glucan is a cell wall constituent specific to fungi and to members of the phylum Stramenopiles, thereby representing a potential MAMP. Despite its abundance, studies focusing on the possible role of β-glucan as a MAMP are rare and the mechanisms of β-glucan perception and signaling in plants are largely unknown. Most plant hosts secrete a large plethora of different hydrolytic enzymes. These include chitinases and glucanases that target fungal cell wall constituents to impact cell wall integrity and to release MAMPs. Direct mechanisms to evade β-glucan recognition by plant-associated fungi have not yet been reported but considering the widespread presence of β-glucan at the fungal cell wall, it is conceivable that plant-associated fungi have evolved sophisticated mechanisms to protect their glucan fibrils from hydrolysis and detection.

The root endophyte *Piriformospora indica* (Basidiomycota, Sebacinales) is a fungal symbiont that colonizes inter- and intracellularly the root epidermal and cortex cells of a broad range of unrelated plants, including the monocotyledonous cereal crop *Hordeum vulgare* (barley) and the dicotyledonous plant *Arabidopsis thaliana,* with a biphasic lifestyle. The initial biotrophic phase is followed by a stage where the fungus is more often found in dead or dying host cells secreting a large variety of hydrolytic enzymes that degrade plant cell walls and proteins. This is especially evident in barley where the symbiont rapidly undergoes a nutritional switch to saprotrophy that correlates with nitrogen limitation and is associated with the production of thinner hyphae in dead root cells (Lahrmann U, Ding Y, Banhara A, Rath M, Hajirezaei MR, Dohlemann S, et al. Host-related metabolic cues affect colonization strategies of a root endophyte. Proc Natl Acad Sci U S A 2013, 110(34): 13965-13970).

Several carbohydrate binding motifs of P. indica proteins are known. P. indica proteins usually contain either one or a combination of the following carbohydrate binding motifs: LysM, WSC or CBM1. LysM is known as a widely distributed protein motif for binding to (peptido)glycans (Buist G et al., (2008) LysM, a widely distributed protein motif for binding to (peptido)glycans. Mol Microbiol 68: 838-847). In fungi, the LysM domains are mainly associated with hydrolytic enzymes acting on fungal cell wall, but they are also present in proteins lacking other conserved domains. A lectin-like LysM protein from Cladosporium fulvum was found to inhibit chitin oligosaccharide triggered and PRR-mediated activation of host immunity (de Jonge R,et al. (2010) Conserved fungal LysM effector Ecp6 prevents chitin-triggered immunity in plants. Science 329: 953-955). However, rather little is known about the function of WSC or CBM1 containing proteins. They were first described in yeast as cell wall integrity sensors involved in mediating intracellular responses to environmental stress (Ponting CP et al. (1999) A latrophilin/CL-1-like GPS domain in polycystin-1. Curr Biol 9: R585-588; Cohen-Kupiec R et al. (1999) Molecular characterization of a novel beta-1,3-exoglucanase related to mycoparasitism of Trichoderma harzianum. Gene 226: 147-154; Verna J et al., (1997) A family of genes required for maintenance of cell wall integrity and for the stress response in Saccharomyces cerevisiae. Proc Natl Acad Sci U S A 94: 13804-13809). The CBM1 domain has cellulose-binding function and is almost exclusively found in fungal hydrolytic enzymes acting on plant cell walls (Boraston AB et al., (2004) Carbohydrate-binding modules: fine-tuning polysaccharide recognition. Biochem J 382: 769-781).

Piriformospora indica has been shown to be good model for studying fungal transcriptional responses associated with the colonization of living and dead barley roots. Studies by Zuccaro et al. showed that about 10% of the fungal genes induced during the biotrophic colonization encoded putative small secreted proteins (SSPs, <300 amino acids), including several lectin-like proteins and members of a P. indica-specific gene family (DELD) (Zuccaro et al.: PLoS Pathog. 2011 Oct; 7(10): e1002290). However, specific fungal effectors that target conserved recognition and signaling pathways in different hosts, i.e. polypeptides that specifically bind certain glucose moieties, i.e. beta-1,6 glucans, have not been characterized.

Lectins and lectin-like proteins that are highly specific for certain sugar moieties are widely used in molecular biology, biochemistry or biomedicine. Wheat germ agglutinine (WGA) is one of the lectins most commonly used in cell biology. For example, a conjugate of WGA and a fluorescent dye is available from Thermo Fisher Scientific Inc. (Catalogue Number #W11261). However, specific conjugates of a beta 1,6-glucan binding polypeptide and a detectable label that could be used for diverse applications in molecular biology, biochemistry or biomedicine, for example in a method for diagnosing a fungal infection in a subject, are currently not available.

There is thus the need to provide novel polypeptides specifically binding to beta 1,6-glucans and especially for conjugates of beta 1,6-glucan binding polypeptide linked to a detectable label that could be used for molecular and/or diagnostic purposes.

The technical problem underlying the present invention can be seen as the provision of means and methods for complying with the aforementioned needs. The technical problem is solved by the embodiments characterized in the claims and herein below.

### Brief Summary of the present invention

The present invention relates to a conjugate comprising i) a beta 1,6-glucan binding polypeptide, or a beta 1,6-glucan binding fragment of said polypeptide, and ii) a detectable label.

In an embodiment of the conjugate, the detectable label is covalently bound to said beta 1,6-glucan binding polypeptide, or said beta 1,6-glucan binding fragment thereof, or the detectable label is non-covalently bound to said a beta 1,6-glucan binding polypeptide, or a beta 1,6-glucan binding fragment thereof.

In an embodiment of the present invention, the detectable label is selected from the group consisting of expressed fluorescent proteins, fluorescent peptide or protein labels, fluorescent dyes, inorganic nanocrystals or nanoparticles with unique optical and chemical properties, chromogens, luminescent or chemiluminescent compounds, radioactive compounds, a combination of an enzyme and its substrate, amino acids, peptides or antibodies linked to signal producing substances, a liposome or vesicle containing signal producing substances and electroactive species.

In an embodiment of the present invention, the detectable label is a peptide or polypeptide, and wherein said conjugate is a fusion polypeptide comprising the beta 1,6-glucan binding polypeptide, or the beta 1,6-glucan binding fragment thereof, and said detectable label.

In an embodiment of the conjugate of the present invention, the beta 1,6-glucan binding polypeptide comprises an amino acid sequence as shown in any one of SEQ ID NO: 2 and 8 to 35 (i.e. in SEQ ID NO: 2, 8, 9, 10, 11, 12, 13, 14, 15, 16, 17, 18, 19, 20, 21, 22, 23, 24, 25, 26, 27, 28, 29, 30, 31, 32, 33, 34, or 35), or is a beta 1,6-glucan binding variant thereof. Preferably, the beta 1,6-glucan binding polypeptide comprises an amino acid sequence as shown in SEQ ID NO: 2 or is a variant thereof.

The present invention also relates to a fragment of a beta 1,6-glucan binding polypeptide or variant thereof, wherein said beta 1,6-glucan binding polypeptide has an amino acid sequence as shown in any one of SEQ ID NOs: 2 and 8 to 35, and wherein the fragment specifically binds to beta 1,6 glucans. Said fragment can be operably linked to a detectable label. Said fragment shall have a length of at least 20 amino acids. Preferably, however the fragment is shorter than the respective full-length polypeptide (as shown in any one of SEQ ID NOs: 2 and 8 to 35).

The present invention also relates to a vector comprising a nucleic acid encoding for a beta 1,6-glucan binding polypeptide of the present invention, or the beta 1,6-glucan binding fragment of the present invention, or the fusion protein as defined herein, i.e. a fusion polypeptide of i) a beta 1,6-glucan binding polypeptide as defined herein, or the beta 1,6-glucan binding fragment thereof and ii) and polypeptide or peptide which is a detectable label.

In an embodiment of the vector, the nucleic acid comprised by said vector is operably linked to a heterologous expression control sequence.

The present invention also concerns a host cell (such as a non-human host cell), or non-human organism comprising a nucleic acid, wherein the nucleic acid is encoding for the beta 1,6-glucan binding polypeptide as defined herein, in particular the 1,6-glucan binding polypeptide of the present invention, or a beta 1,6-glucan binding fragment of the present invention, the fusion protein as defined herein, or the vector of the present invention.

The present invention also relates to the use of the conjugate of the present invention, the fragment of the present invention, the 1,6-glucan binding polypeptide of the present invention, the vector of the present invention for detecting beta 1,6 glucans and/or fungal cells in a sample, or for diagnosing fungal infection in a sample.

Further encompassed by the present invention is a method for the detection of beta 1,6 glucans and/or fungal cells in a sample, said method comprising, providing a sample suspected to comprise beta 1,6 glucans and/or fungal cells, and contacting said sample with the conjugate of the present invention, the polypeptide of the present invention, the fragment of the present invention, the vector of the present invention or the host cell non-human organism of the present invention.

The present invention also relates to a method for diagnosing fungal infection in a subject, said method comprising, providing a sample from a subject suspected to have a fungal infection, contacting said sample with the conjugate of the present invention, the polypeptide of the present invention, or the fragment of the present invention. Thereby, the presence or absence of fungal infection in said subject is diagnosed.

The present invention further relates to a beta 1,6-glucan binding polypeptide, wherein said polypeptide comprises an amino acid sequence as shown in any one of SEQ ID NO: 2 and 8 to 35, or wherein said polypeptide is a beta 1,6-glucan binding variant of the polypeptide comprising an amino acid sequence as shown in any one of SEQ ID NO: 2 and 8 to 35.

The present invention further relates to a polynucleotide encoding the beta 1,6-glucan binding fragment, or the beta 1,6-glucan binding polypeptide of the present invention, or a fusion protein as defined herein (in connection with the definition of the conjugate of the present invention). Further, the present invention relates to a variant of said polynucleotide.

The present invention further relates to a complex comprising
i) at least one beta 1,6 glucan, and
ii) the polypeptide of the present invention, the fragment of the present invention, or the conjugate of the present invention.

Further the present invention relates to an aptamer and, in particular, an antibody which specifically binds to the polypeptide or fragment of the present invention.

Further, the present invention relates to a conjugate comprising the beta 1,6-glucan binding fragment or beta 1,6-glucan binding polypeptide of the present invention.

Further encompassed by the present invention is a kit comprising the conjugate of the present invention, the polypeptide of the present invention, the polynucleotide of the present invention, the antibody of the present invention, the fragment of the present invention, the vector of the present invention or the host cell or non-human organism of the present invention.

### Detailed Summary of the present invention - Definitions

As set forth above, the invention relates to a conjugate comprising i) a beta 1,6-glucan binding polypeptide, or a beta 1,6-glucan binding fragment of said polypeptide, and ii) a detectable label.

The term "detectable label" as used herein refers to any label that can be detected by any means. A detection signal can be produced by any kind of substance, for example by a luminescent compound, a fluorescent protein or a combination of an enzyme and a substrate. P

Preferably, the detectable label is a visual label. The term "visual label" as used herein preferably refers to a label that is visually detectable, i.e. by detections of (emitted) photons.

Means and methods to detect labels and especially visual labels are known in the art and include, for example, spectrophotometry, fluorescence microscopy, bright-field microscopy or single photon emission computed tomography. Preferably, the detectable label is selected from the group consisting of: a fluorescent protein (such as Green Fluorescent Protein (GFP), Cyan Fluorescent Protein (CFP), Red Fluorescent Protein (RFP), a fluorescent peptide, a fluorescent protein label (Allophycocyanin (APC), R-Phycoerythrin (R-PE)), a fluorescent dye, an inorganic nanocrystal (in particular with unique optical and chemical properties) or a nanoparticle (in particular with unique optical and chemical properties), a chromogen, a luminescent label, a radioactive label, an enzyme such as horseradish peroxidase or alkaline phosphatase), a substrate of an enzyme, biotin, an amino acids, a peptide or an antibody which specifically binds to the beta 1,6-glucan binding polypeptide, or the beta 1,6-glucan binding fragment, a liposome or vesicle containing signal producing substances and electroactive species. More preferably, the detectable label is a fluorescent label. In particular, said fluorescent label is selected from the group consisting of Green Fluorescent Protein (GFP), Cyan Fluorescent Protein (CFP), Red Fluorescent Protein (RFP), Allophycocyanin (APC), R-Phycoerythrin (R-PE), and a fluorescent dye. Preferably, the fluorescent dye is selected from Alexa Fluor® 350, Alexa Fluor® 405, Alexa Fluor® 488, Alexa Fluor® 532, Alexa Fluor® 546, Alexa Fluor® 555, Alexa Fluor® 568, Alexa Fluor® 594, Alexa Fluor® 647, Alexa Fluor® 680, Alexa Fluor® 750, BODIPY® FL, Coumarin, Cy®3, Cy®5, Fluorescein (FITC), Oregon Green®, Pacific Blue™, Pacific Green™, Pacific Orange™, Tetramethylrhodamine (TRITC), Texas Red®, Odot® 525, Odot® 565, Odot® 605, Odot® 655, Odot® 705, Odot® 800, ATTO 390, ATTO 425, ATTO 465, ATTO 488, and ATTO 495 or is a variant of any of the aforementioned labels.

The term "variant" as used herein in conjunction with said "fluorescent label" refers to a variant of said fluorescent label that is still able to exert its function, in essence to emit fluorescence at a predetermined wave length spectrum, also referred to as emission spectrum, after an irradiation at a predetermined wave length spectrum, also known as excitation spectrum. Such a variant could be, for example, a polypeptide variant of the green fluorescence protein which contains one or more amino acid substitutions or a variant of a fluorescent dye which contains an additional chemical residue.

Preferably, the detectable label is heterologous with respect to the beta 1,6-glucan binding polypeptide, or the beta 1,6-glucan binding fragment of said polypeptide. Further, it is envisaged that the detectable label is conjugated with, i.e. operably linked to the fragment or polypeptide. The operable linkage shall allow for the detection of beta 1,6-glucans with the conjugate of the present invention. E.g. the linkage can be achieved by covalent bindings or non-covalent bindings between the detectable label and the beta 1,6-glucan binding polypeptide, or the beta 1,6-glucan binding fragment of said polypeptide.

Thus, in a preferred embodiment of the present invention, the detectable label is covalently bound to the beta 1,6-glucan binding polypeptide, or the beta 1,6-glucan binding fragment thereof. E.g, the beta 1,6-glucan binding polypeptide, or the beta 1,6-glucan binding fragment thereof, and the detectable label comprised in the conjugate may be covalently bound to each other by a peptide bond or peptide bonds.

In another preferred embodiment of the present invention, the detectable label is non-covalently bound to the beta 1,6-glucan binding polypeptide, or the beta 1,6-glucan binding fragment thereof. Preferably, non-covalent bindings include binding by disulfide bonds, affinity binding based on ionic bonds, binding by hydrogen bonds and/or binding by van der Waals forces.

It is to be understood that the detectable label is heterologous with respect to the beta 1,6-glucan binding polypeptide, or the beta 1,6-glucan binding fragment thereof.

Further, it is envisaged that the binding of the detectable label to the polypeptide or fragment in the conjugate does not affect, in particular does not effect significantly, the capability of the fragment or polypeptide to bind beta 1,6 glucans.

The detectable label can be linked with the polypeptide or fragment after synthesis of the polypeptide or the fragment, for example by modifying isolated peptides. Alternatively, the detectable label can be linked with the polypeptide or fragment during peptide synthesis, in particular during translation. For example, a fusion polypeptide comprising the beta 1,6-glucan binding polypeptide or the beta 1,6-glucan binding fragment thereof and a fluorescent polypeptide such as the Green fluorescent protein (GFP) can be produced by translation.

As set forth above, the detectable label is preferably a fluorescent label. Fluorescent labels are well known in the art. Preferably, a "fluorescent label" is a substance which emits fluorescence as a result of irradiation of a predetermined excitation light or excitation using field effect. Fluorescence includes various kinds of emission such as phosphorescence. Suitable expressed fluorescent proteins include, for example, Green Fluorescent Protein (GFP), Cyan Fluorescent Protein (CFP) or Red Fluorescent Protein (RFP). Fluorescent peptide or protein labels include, for example, Allophycocyanin (APC) or R-Phycoerythrin (R-PE). Fluorescent dyes include, for example, fluorescent dyes of the fluorescein family (manufactured by Integrated DNA Technologies), fluorescent dyes of the polyhalofluorescein family (manufactured by Applied Biosystems) fluorescent dyes of the hexachlorofluorescein family (manufactured by Applied Biosystems) fluorescent dyes of the coumarin family (manufactured by Invitrogen), fluorescent dyes of the rhodamine family (manufactured by GE Healthcare Bioscience), fluorescent dyes of the cyanine family, fluorescent dyes of the indocarbocyanine family, fluorescent dyes of the oxazine family, fluorescent dyes of the thiazine family, fluorescent dyes of the squaraine family, fluorescent dyes of the chelated lanthanide family, fluorescent dyes of the BODIPY (registered trademark) family (manufactured by Invitrogen), fluorescent dyes of the naphthalenesulfonic acid family, fluorescent dyes of the pyrene family, fluorescent dyes of the triphenylmethane family, Alexa Fluor (registered trademark) dye series (manufactured by Invitrogen), Atto dye series (manufactured by Atto-Tec GmbH), inorganic nanocrystals or nanoparticles with unique optical and chemical properties include, for example, Qdot (registered trademark) probes or gold nanoparticles. Encompassed as detectable labels are also chromogens which form color when oxidized such as permanent chromogens, i.e. for use with commonly used enzyme labels such as Horseradish Peroxidase (HRP) and Alkaline Phosphatase (AP), luminescent or chemiluminescent compounds such as 4-Aminophthalhydrazide, radioactive compounds such as ³⁵S labeled amino acids, a combination of an enzyme and its substrate such as the enzyme alkaline phosphatase combined with a respective substrate, amino acids, peptides or antibodies linked to signal producing substances, for example antibodies or biotinylated amino acid linked to an enzyme in combination with a respective substrate, a liposome or vesicle containing signal producing substances and electroactive species that can be used for a light-based readout system.

In a preferred embodiment, the detectable label is selected from a fluorescent label, a magnetic label (e.g. magnetic beads, including paramagnetic and superparamagnetic labels), a radioactive label, an enzymatically active label, gold particles, latex beads, acridan ester, luminol, and ruthenium. Enzymatically active labels include e.g. horseradish peroxidase, alkaline phosphatase, beta-Galactosidase, and Luciferase. Typical radioactive labels include 35S, 125I, 32P, 33P and the like. A radioactive label can be detected by any method known and appropriate, e.g. a light-sensitive film or a phosphor imager.

Further, the detectable label may be also a "tag". Thus, in embodiment the detectable label is seletected from biotin, digoxygenin, His-Tag, Glutathion-S-Transferase, FLAG, myc-tag, influenza A virus haemagglutinin (HA), and maltose binding protein.

Permanent binding between the beta 1,6-glucan binding polypeptide, or the beta 1,6-glucan binding fragment thereof, and the detectable label, can for example be achieved by recombinant manufacture. To this end, a polynucleotide encoding beta 1,6-glucan binding polypeptide, or the beta 1,6-glucan binding fragment thereof and a detectable label, for example GFP, is synthesized either chemically or by recombinant DNA techniques and expressed in a suitable expression system. Afterwards, the expressed fusion polypeptide comprising the beta 1,6-glucan binding polypeptide, or the beta 1,6-glucan binding fragment thereof, and the detectable label can be purified from the expression system.

Green Fluorescent Protein (GFP), Cyan Fluorescent Protein (CFP), Red Fluorescent Protein (RFP) are fluorescent proteins. in a preferred the detectable label is a peptide or polypeptide such as a fluorescent protein. If the detectable label is a protein or peptide, the conjugate is a fusion polypeptide comprising the beta 1,6-glucan binding polypeptide, or the beta 1,6-glucan binding fragment thereof, and said peptide or polypeptide (such as GFP).

Various affinity binding systems comprising a ligand and a receptor portion are known in the art such that the skilled artisan can be used in order to construe fusion polypeptides comprising the beta 1,6-glucan binding polypeptide, or the beta 1,6-glucan binding fragment thereof, and the detectable label non-covalently bound to each other without further ado. In an embodiment, the binding system is based on antibody/antigen interaction or streptavidin/biotin interaction, or avidin/biotin interaction.

The conjugate according to the present invention may also comprise further components such as linker or spacer sequence between the beta 1,6-glucan binding polypeptide, or the beta 1,6-glucan binding fragment thereof and the detectable label. Moreover, the conjugate may comprise components contributing further functions such as components which increase the stability of the conjugate as well as component which facilitate manufacture and/or purification of the conjugate such as tags.

The conjugate may be used for diverse purposes including various molecular, biochemical, biomedical and diagnostic methods such as Histochemistry, enzyme-linked immunosorbent assays (ELISA) or Fluorescent in situ Hybridization (FISH).

The terms "polypeptide" and "peptide" are well known in the art. As used herein, the term "polypeptide" preferably refers to a molecule comprising at least 20, at least 30, at least 40, at least 50 or at least 60 amino acids that are covalently linked to each other by peptide bonds. A peptide preferably comprises less than 20 amino acids covalently linked by peptide bonds.

The "beta 1,6-glucan binding polypeptide" or "beta 1,6-glucan binding fragment thereof" according to the present invention shall bind to specific sugar moieties. Preferably, the 1,6-glucan binding polypeptide or the beta 1,6-glucan binding fragment thereof shall bind to beta 1,6-glucans. In particular, it shall bind specifically to beta 1,6-glucans.

Beta 1,6-glucans are well known in the art and e.g. described in Fesel and Zuccaro (β-glucan: Crucial component of the fungal cell wall and elusive MAMP in plants. Fungal Genetics and Biology. Volume 90, May 2016, Pages 53-60) which herewith is incorporated by reference in its entirety.

The term β-glucans used within the meaning of the present invention relates to glucans in which mainly glucose units are linked via beta-glycosidic linkages.

The term 1,6 β-glucans (1.6-beta-glucans) within the meaning of the present invention refers to glucans comprising at least one 1,6-glycosidic linkage, i.e. an 1,6-beta glycosidic linkage between two D-glucose units. Preferred 1,6-β-glucans comprise at least one 1,6 glycosidic bond and consist of D-glucose units, preferably linked with each other only via beta glycosidic bonds.

The term "a beta 1,6-glucan binding polypeptide" is consequently denoted to mean a polypeptide which binds to glucans comprising at least one 1,6-beta glycosidic bond. Thus, a beta 1,6-glucan binding fragment of said polypeptide is consequently denoted to mean a fragment (of said polypeptide) which binds to glucans comprising at least one 1,6-beta glycosidic bond. Preferably, the beta 1,6-glucan binding polypeptide or the beta 1,6-glucan binding fragment thereof does not bind to glucans which do not comprise such at least one 1,6-beta-glycosidic bond. In an embodiment, the polypeptide or fragment can have increased binding specificity to beta 1,6 glycosidic bonds in context on non-binding glucan background.

The glucan is preferably a beta glucan. The at least one 1,6-beta glycosidic bond comprised by the beta glucan shall be present in a context of a β-1,3- or β-1,2 glucan backbone or similar. Thus, the beta 1,6-glucan binding polypeptide or fragment (or variant) shall bind beta glucan via beta-1,6-linked glucose moieties, preferably, in a context of a β-1,2 glucan backbone or, in particular, a beta-1,3 glucan backbone.

Thus, it is envisaged that the polypeptide/fragment shall bind to beta-1,6-glucan which is covalently bound to beta 1,3 glucan (or beta 1,2 glucan), in particular in the form of branching polysaccharides.

In an embodiment of the present invention, the beta 1,6-glucan binding polypeptide comprises an amino acid sequence as shown in any one of SEQ ID NO: 2 and 8 to 35, or is a beta 1,6-glucan binding variant thereof. Said variant shall be a beta 1,6-glucan binding variant.

In a preferred embodiment of the present invention, the beta 1,6-glucan binding polypeptide comprises an amino acid sequence as shown in SEQ ID NO: 2 or is a variant thereof, i.e. a beta 1,6 glucan binding variant thereof. Preferably, the polypeptide comprising an amino acid sequence as shown in SEQ IN NO: 2 is encoded by a polynucleotide comprising a sequence as shown in SEQ ID NO: 1.

The present invention also relates to a fragment of a beta 1,6-glucan binding polypeptide or variant thereof, wherein said beta 1,6-glucan binding polypeptide has an amino acid sequence as shown in any one of SEQ ID NOs: 2 and 8 to 35, and wherein the fragment binds to beta 1,6 glucans. Said fragment can be operably linked to a detectable label.

In a particular preferred embodiment of the fragment of the present invention, the fragment is beta 1,6-glucan binding fragment of the beta 1,6-glucan binding polypeptide having an amino acid as shown in SEQ ID NO: 2, or is a beta 1,6-glucan binding variant of a fragment of the beta 1,6-glucan binding polypeptide having an amino acid as shown in SEQ ID NO: 2.

It is to be understood that a fragment of a polypeptide is shorter than the full length amino acid sequences of the amino acid sequence as shown in any one of SEQ ID NOs: 2 and 8 to 35. E.g. with respect to SEQ ID NO: 2, a fragment of the polypeptide having an amino acid as shown in SEQ ID NO: 2 shall be shorter than the this polypeptide. Accordingly, the fragment has a length of less than 104 amino acids (such as a length of less than 100 amino acids).

Preferably, the beta 1,6-glucan binding fragment has a length of at least 15 amino acids, in particular of at least 20 amino acids. More preferably, said fragment shall have a length of at least 30 amino acids. In further embodiments, the fragment shall have a length of at least 40, at least 50 or at least 60 amino acids.

Also preferably, the length of beta 1,6-glucan binding fragment shall not exceed a length of 80 amino acids. E.g. it is envisaged that the length of beta 1,6-glucan binding fragment shall not exceed a length of 70 amino acids. Thus, the fragment can e.g. have a length of at least 20 acids to 80 amino acids, or of 30 to 70 amino acids.

The fragment shall comprise consecutive amino acids of the respective full length polypeptide, e.g. of the polypeptide having a sequence as shown in SEQ ID NO: 2.

In a preferred embodiment, the fragment of the present invention is a fragment comprised by the C-terminal portion of the beta 1,6-glucan binding polypeptide having a sequence as shown in SEQ ID NO: 2. In particular, the fragment comprises the C-terminal end of the beta 1,6-glucan binding polypeptide having a sequence as shown in SEQ ID NO: 2.

E.g., it is envisaged that the beta 1,6-glucan binding fragment comprises at least 15, at least 20, at least 30, at least 40, at least 50 or at least 60 consecutive amino acids comprised in the C-terminal portion of the beta 1,6-glucan binding polypeptide as shown in SEQ ID 2 (or is a variant thereof). In particular, the fragment shall comprise at least 15, at least 20, at least 30, at least 40, at least 50 or at least 60 consecutive amino acids of the C-terminal end of the beta 1,6-glucan binding polypeptide having a sequence as shown in SEQ ID NO: 2 (or is a variant thereof). E.g. the fragment preferably comprises (or consists of) in increasing order of preference the following consecutive amino acids of SEQ ID NO: 2; the amino acids from position 90 to 104, the amino acids from position 85 to 104, more preferably the amino acids from position 75 to 104, even more preferably the amino acids from position 65 to 104, and most preferably the amino acids from position 55 to 104 of SEQ ID NO:2. Moreover, it may comprise (or consist of) the amino acids from position 48 to 104 of SEQ ID NO:2. Further fragments envisaged by the present invention are variants of the aforementioned fragments.

As stated elsewhere the double C99A C104A mutant of FGB1 is still biologically active. Therefore, in an embodiment it is envisaged that the fragment of beta 1,6-glucan binding polypeptide having a sequence as shown in SEQ ID NO: 2 does not comprise the last five, or in particular the last six amino acids of the polypeptide having a sequence as shown in SEQ ID NO: 2.

Accordingly, that the fragment preferably might comprise (or may consist of) in increasing order of preference the following consecutive amino acids of SEQ ID NO: 2; the amino acids from position 84 to 98, the amino acids from position 79 to 98, more preferably the amino acids from position 69 to 98, even more preferably the amino acids from position 59 to 98, and most preferably the amino acids from position 49 to 98 of SEQ ID NO:2. Moreover, it may comprise (or consist of) the amino acids from position 48 to 98 of SEQ ID NO:2. Further fragments envisaged by the present invention are variants of the aforementioned fragments.

The N-terminal end of the polypeptide having a sequence as shown in SEQ ID NO; 2 appears to be irrelevant for the binding. Therefore, it is contemplated that the fragment thereof does not comprise the 30 N-terminal amino acids (i.e. consecutive amino acids), in particular does not comprise at the 30 N-terminal amino acids of the polypeptide having a sequence as shown in SEQ ID NO; 2.

Encompassed by the present invention are also variants of the 1,6-glucan binding polypeptide, or beta 1,6-glucan binding fragment thereof. Thus, the term "1,6-glucan binding polypeptide" preferably also encompasses variants of the said 1,6-glucan binding polypeptide. Also, the term "1,6-glucan binding fragment" preferably also encompasses variants of the said 1,6-glucan binding fragment. Such a variant, typically, differs from the specific amino acid sequences referred to before by at least one amino acid substitution, deletion and/or addition. Nevertheless, the variant of 1,6-glucan binding polypeptide, or beta 1,6-glucan binding fragment thereof shall still be able to exert its function, in essence the capability to bind to 1,6-glucans. The variant of a beta 1,6-glucan binding polypeptide shall, preferably, comprise (or have) an amino acid sequence which is at least 50%, at least 60 %, at least 70%, at least 75%, at least 80%, at least 85%, at least 90%, at least 95%, at least 96%, at least 97%, at least 98% or at least 99% identical to the amino acid sequence of a 1,6-glucan binding polypeptide as referred to herein, more preferably to an amino acid sequence as shown in SEQ ID NO: 2 to 8 to 35 (i.e. SEQ ID NO: 2, 8, 9, 10, 11, 12, 13, 14, 15, 16, 17, 18, 19, 20, 21, 22, 23, 24, 25, 26, 27, 28, 29, 30, 31, 32, 33, 34, or 35), or to an amino acid sequence encoded by the nucleic acid sequences shown in SEQ ID NO: 1. The variant of a beta 1,6-glucan binding fragment (or of a conserved domain as referred to herein) shall, preferably, comprise (or have) an amino acid sequence which is at least 50%, at least 60 %, at least 70%, at least 75%, at least 80%, at least 85%, at least 90%, at least 95%, at least 96%, at least 97%, at least 98% or at least 99% identical to a fragment as referred to herein, more preferably to an amino acid sequence of a 1,6-glucan binding polypeptide as referred to herein, and most preferably to a fragment of an amino acid sequence as shown in SEQ ID NO: 2 to 8 to 35 (i.e. SEQ ID NO: 2, 8, 9, 10, 11, 12, 13, 14, 15, 16, 17, 18, 19, 20, 21, 22, 23, 24, 25, 26, 27, 28, 29, 30, 31, 32, 33, 34, or 35), or to an amino acid sequence encoded by the nucleic acid sequences shown in SEQ ID NO: 1. The variant of a of a conserved domain as referred to herein shall, preferably, comprise (or have) an amino acid sequence which is at least 50%, at least 60 %, at least 70%, at least 75%, at least 80%, at least 85%, at least 90%, at least 95%, at least 96%, at least 97%, at least 98% or at least 99% identical to the amino acid sequence of the conserved domain.

Sequence identity between amino acid sequences or nucleic acid sequences as used herein can be assessed by determining the number of identical nucleotides or amino acids between two nucleic acid sequences or amino acid sequences wherein the sequences are aligned so that the highest order match is obtained. It can be calculated using published techniques or methods codified in computer programs such as, for example, BLASTP, BLASTN or FASTA (Altschul 1990, J Mol Biol 215, 403). The percent identity values are, preferably, calculated over a comparison window. A comparison window, preferably, is the length of the entire sequence of the shorter sequence to be aligned or at least half of said sequence. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (Higgins 1989, CABIOS 5, 151) or the programs Gap and BestFit (Needleman 1970, J Mol Biol 48: 443; Smith 1981, Adv Appl Math 2: 482), which are part of the GCG software packet (Genetics Computer Group 1991, 575 Science Drive, Madison, Wisconsin, USA 53711), may be used. The sequence identity values recited above in percent (%) are to be determined, in another aspect of the invention, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

Preferably, the beta 1,6-glucan binding polypeptide (or variant thereof), or beta 1,6-glucan binding fragment (or variant thereof), comprises a conserved domain having the amino acid sequence ACxASxSCNCSGlkxGLFCGDGlfNCKKGHVYQCxtDGhxTCDYGVRTSCQKCnKLSC or a variant of said domain,
wherein X is, preferably, any amino acid. The sequence is shown in SEQ ID NO: 3.

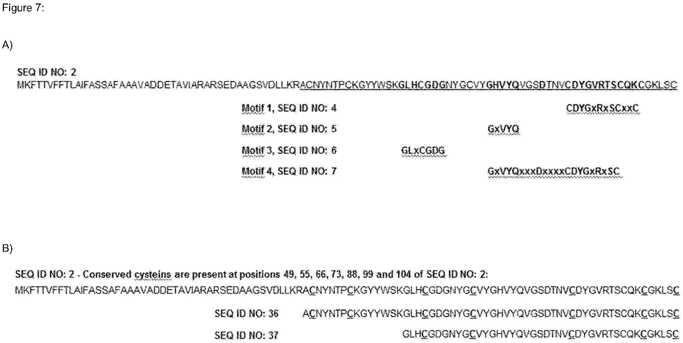

Alternatively, it is envisaged that the beta 1,6 glucan binding polypeptide (or variant thereof) or fragment (or variant thereof) comprises an amino acid sequence as shown in SEQ ID NO: 37 or a variant thereof (such as a sequence which is at least 50% identical thereto), or in particular as shown in SEQ ID NO: 36 or variant thereof (such as a sequence which is at least 50% identical thereto). The sequences of these conserved domains are shown in Figure 7B.

Moreover, it is envisaged that the beta 1,6-glucan binding polypeptide (or variant thereof), or beta 1,6-glucan binding fragment thereof (or variant thereof) comprises one, two, three or four motifs of Motifs 1 to 4:
Motif 1 has an amino acid sequence as shown in SEQ ID NO: 4 (CDYGxRxSCxxC)
Motif 2 has an amino acid sequence as shown in SEQ ID NO: 5 (GxVYQ)
Motif 3 has an amino acid sequence as shown in SEQ ID NO: 6 (GLxCGDG)
Motif 4 has an amino acid sequence as shown in SEQ ID NO: 7
(GxVYQxxxDxxxxCDYGxRxSC)

Again, "X" in motifs 1 to 4 is, preferably, any amino acid.

The position of motifs 1 to 4 in the polypeptide having an amino acid sequence as shown in SEQ ID NO: 2 is shown in Figure 7A.

Preferably, the beta 1,6-glucan binding polypeptide (or variant thereof), or beta 1,6-glucan binding fragment thereof (or variant thereof) comprises Motif 1, more preferably, it comprises Motifs 1 and 2, even more preferably motifs 1 and 3, and most preferably Motifs 1, 2 and 3.

Further preferably, it comprises motif 4. In particular, it comprise Motifs 3 and 4. Moreover, it is envisaged that it comprises all four motifs.

The order of the motifs, from N- to C-terminal is preferably as shown in Figure 7A. Thus, if the beta 1,6-glucan binding polypeptide (or variant thereof), or beta 1,6-glucan binding fragment thereof (or variant thereof) comprises Motifs 1 to 3, the order is preferably as follows (from the N-terminus to the C-terminus) or the polypeptide or fragment:
Motif 3 - Motif 2 - Motif 1.

In an embodiment of Motif 1, Motif 1 has an amino acid sequence as shown in SEQ ID NO: 40 (CDYGVRTSCQK)

In an embodiment of Motif 1, Motif 2 has an amino acid sequence as shown in SEQ ID NO: 41 (GHVYQ)

In an embodiment of Motif 3, Motif 3 has an amino acid sequence as shown in SEQ ID NO: 42 (GLHCGDG)

In an embodiment of Motif 4, Motif 4 has an amino acid sequence as shown in SEQ ID NO: 43
(GHVYQVGSDTNVCDYGVRTSCQK)

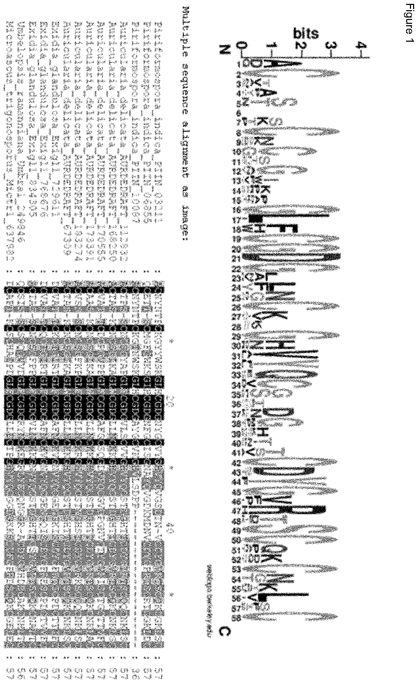

A sequence alignment carried out in the context of the present invention, showed that the polypeptides as referred herein comprise conserved cysteine residues at positions 49, 55, 66, 73, 88, 99 and 104 of SEQ ID NO: 2 (see Figure 1 and 7B). Accordingly, the beta 1,6-glucan binding polypeptide (or variant thereof), or beta 1,6-glucan binding fragment thereof (or variant thereof), or the conserved domain (or variant thereof) preferably comprises (conserved) cysteine residues at the position corresponding to position 49, 55, 66, 73, 88, 99 and 104 of SEQ ID NO: 2.

Experiments that were carried out in the context of the present invention showed that the double C99A C104A mutant of FGB1 (i.e. the polypeptide having a sequence as shown in SEQ ID NO: 2) is still biologically active. Thus, not all conserved cysteine residues as referred to in the previous paragraph would have to present in the polypeptide/fragment/variant of the present invention.

In an embodiment, the beta 1,6-glucan binding polypeptide (or variant thereof), or beta 1,6-glucan binding fragment thereof (or variant thereof), or the conserved domain (or variant thereof) comprises (conserved) cysteine residues at the position corresponding to position 49, 55, 66, 73, and 88 of SEQ ID NO: 2. In a further embodiment, it comprises cysteine residues at the position corresponding to position 66, 73, 88, and 99 of SEQ ID NO: 2 In an even further embodiment, it comprises cysteine residues at the position corresponding to position 55, 66, 73, and 88 of SEQ ID NO: 2.

Thus, a variant of beta 1,6-glucan binding polypeptide preferably comprises an amino acid sequence which is at least 50%, at least 60 %, at least 70%, at least 75%, at least 80%, at least 85%, at least 90%, at least 95%, at least 96%, at least 97%, at least 98% or at least 99% identical to SEQ ID NO: 2. In addition, said polypeptide may comprise
a) a conserved domain having an amino acid sequence as shown in SEQ ID NO: 3, 36 or 37 or an amino acid sequence which is at least 50%, at least 60 %, at least 70%, at least 75%, at least 80%, at least 85%, at least 90%, at least 95%, at least 96%, at least 97%, at least 98% or at least 99% identical to SEQ ID NO: 3, 36, or 37,
b) one, two, three or four motifs of Motifs 1 to 4 as described above, and/or
c) conserved Cysteine residues as described above.

A fragment as referred herein (such as a fragment of SEQ ID NO: 2) preferably comprises
a) an amino acid sequence as shown in SEQ ID NO: 3, 36 or 37 or an amino acid sequence which is at least 50%, at least 60 %, at least 70%, at least 75%, at least 80%, at least 85%, at least 90%, at least 95%, at least 96%, at least 97%, at least 98% or at least 99% identical to SEQ ID NO: 3, 36, or 37,
b) one, two, three or four motifs of Motifs 1 to 4 as described above, and/or
c) conserved Cysteine residues as described above.

Preferably, said fragment is shorter than 104 amino acids.

Moreover, the invention also encompasses a vector comprising a nucleic acid encoding for the beta 1,6-glucan binding polypeptide or a variant as set forth herein, or beta 1,6-glucan binding fragment as set forth herein (or a variant thereof) or a fusion polypeptide comprising the beta 1,6-glucan binding polypeptide, or the beta 1,6-glucan binding fragment thereof, and the detectable label. In particular, the present invention relates to a vector comprising a nucleic acid encoding for a beta 1,6-glucan binding polypeptide, or the beta 1,6-glucan binding fragment of the present invention, or the fusion protein as defined herein, i.e. a fusion polypeptide of i) a beta 1,6-glucan binding polypeptide as defined herein, or the beta 1,6-glucan binding fragment thereof and ii) and polypeptide or peptide which is a detectable label.

Preferably, the nucleic acid comprised by said vector is operably linked to a heterologous expression control sequence.

The term "vector", preferably, encompasses phage, plasmid, viral vectors as well as artificial chromosomes, such as bacterial or yeast artificial chromosomes. Moreover, the term also relates to targeting constructs which allow for random or site-directed integration of the targeting construct into genomic DNA. Such target constructs, preferably, comprise DNA of sufficient length for either homologous or heterologous recombination. The vector encompassing the polynucleotide of the present invention, preferably, further comprises selectable markers for propagation and/or selection in a host. The vector may be incorporated into a host cell by various techniques well known in the art. If introduced into a host cell, the vector may reside in the cytoplasm or may be incorporated into the genome. In the latter case, it is to be understood that the vector may further comprise nucleic acid sequences which allow for homologous recombination or heterologous insertion. Vectors can be introduced into prokaryotic or eukaryotic cells via conventional transformation or transfection techniques. The terms "transformation" and "transfection", conjugation and transduction, as used in the present context, are intended to comprise a multiplicity of prior-art processes for introducing foreign nucleic acid (for example DNA) into a host cell, including calcium phosphate, rubidium chloride or calcium chloride co-precipitation, DEAE-dextran-mediated transfection, lipofection, natural competence, carbon-based clusters, chemically mediated transfer, electroporation or particle bombardment. Suitable methods for the transformation or transfection of host cells, including plant cells, can be found in Sambrook et al. (Molecular Cloning: A Laboratory Manual, 2nd ed., Cold Spring Harbor Laboratory, Cold Spring Harbor Laboratory Press, Cold Spring Harbor, NY, 1989) and other laboratory manuals, such as Methods in Molecular Biology, 1995, Vol. 44, Agrobacterium protocols, Ed.: Gartland and Davey, Humana Press, Totowa, New Jersey. Alternatively, a plasmid vector may be introduced by heat shock or electroporation techniques. Should the vector be a virus, it may be packaged in vitro using an appropriate packaging cell line prior to application to host cells.

In order to express a nucleic acid, for example, a nucleic acid encoding for polypeptide, fragment thereof or a fusion polypeptide, the nucleic acid encoding said for polypeptide, fragment thereof or a fusion polypeptide shall be operably linked to one or more expression control sequences. Preferably, the expression control sequences are heterologous with respect to the nucleic acid operably linked thereto. Thus, in an embodiment, the expression control sequence is not the native promoter.

The term "expression control sequence" as used herein refers to a nucleic acid sequence which is capable of governing, i.e. initiating and controlling, transcription of a nucleic acid sequence of interest, in the present case the nucleic sequences recited above. Such a sequence usually comprises or consists of a promoter or a combination of a promoter and enhancer sequences. Expression of a polynucleotide comprises transcription of the nucleic acid molecule, preferably, into a translatable mRNA. Additional regulatory elements may include transcriptional as well as translational enhancers.

Preferably, the promotor shall be active in yeast and fungi. For example, it is envisaged that eh promoter is active in *Pichia Pastoris* or *Echerichia coli.* The following promoters and expression control sequences may, preferably, be used in an expression vector according to the present invention. The cos, tac, trp, tet, trp-tet, Ipp, lac, Ipp-lac, laclq, T7, T5, T3, gal, trc, ara, SP6, λ-PR or λ-PL promoters are, preferably, used in Gram-negative bacteria. For Gram-positive bacteria, promoters such as SPO2 may be used. From yeast or fungal promoters ADC1, AOX1r, GAL1, MFα, AC, P-60, CYC1, GAPDH, TEF, rp28, ADH are, preferably, used. For animal cell or organism expression, the promoters CMV-, SV40-, RSV-promoter (Rous sarcoma virus), CMV-enhancer, SV40-enhancer are preferably used. From plants the promoters CaMV/35S, nos or the ubiquitin promoter can be used.

The vector of the present invention may also comprise additional polynucleotide sequences such as a terminator sequence, which is a nucleic acid sequence capable of terminating transcription. Such terminator sequences will cause dissociation of the transcription machinery from the nucleic acid sequence to be transcribed. In an embodiment, the terminator shall be active in yeast, fungi such as *Pichia Pastoris* or *Echerichia coli.*

For the purposes of the invention "recombinant" as used herein preferably means a nucleic acid sequence or a vector comprising the nucleic acid, a host cell or non-human organism comprising a nucleic acid according to the invention, all those constructions brought about by recombinant methods in which either the nucleic acid sequence, or a genetic control sequence which is operably linked with the nucleic acid sequence, for example a promoter, or are not located in their natural genetic environment or have been modified by recombinant methods.

The term "operatively linked" in connection with expression control sequences means that the expression control sequence and the nucleic acid of interest are linked so that the expression of the said nucleic acid of interest can be governed by the said expression control sequence, i.e. the expression control sequence shall be functionally linked to the said nucleic acid sequence to be expressed. Accordingly, the expression control sequence and, the nucleic acid sequence to be expressed may be physically linked to each other, e.g., by inserting the expression control sequence at the 5' end of the nucleic acid sequence to be expressed. Alternatively, the expression control sequence and the nucleic acid to be expressed may be merely in physical proximity so that the expression control sequence is capable of governing the expression of at least one nucleic acid sequence of interest. The expression control sequence and the nucleic acid to be expressed are, preferably, separated by not more than 500 bp, 300 bp, 100 bp, 80 bp, 60 bp, 40 bp, 20 bp, 10 bp or 5 bp.

Advantageously, it has been found in the studies underlying the present invention that the polypeptide having an amino acid sequence as shown in SEQ ID NO: 2 (internal name: "FGB-1) efficiently binds to beta 1,6-glucans that are present within the cell wall/at the surface of fungi. Thanks to the present invention, a conjugate, fragment, vector, host cell or kit comprising FGB1 ("a beta 1,6-, glucan binding polypeptide or a beta 1,6-glucan binding fragment thereof") as set forth herein can be used for various applications. For example, a conjugate comprising a beta 1,6-, glucan binding polypeptide or a beta 1,6-glucan binding fragment thereof and a detectable (fluorescent) label can be used as "molecular probe" in an in situ fluorescence hybridization assay (FISH) and/or in a method for detecting fungal contaminations and/or diagnosing a fungal infection.

The present invention also concerns a host cell (such as a human or non-human host cell) or non-human organism comprising a nucleic acid, wherein the nucleic acid is encoding for the beta 1,6-glucan binding polypeptide of the present invention, or the beta 1,6-glucan binding fragment of the present invention, the fusion protein as defined herein (in connection with the conjugate), or the vector of the present invention. The present invention also concerns a host cell (such as a or non-human host cell) or non-human organism comprising a nucleic acid, wherein the nucleic acid is the polynucleotide of the present invention.

A host cell in the context of the present invention may be any cell such as a plant cell, or fungal cell. Preferably, the host cell is a bacterial cell such as Escherichia coli, a yeast cell or fungal cell such as a Saccharomyces Cerevisiae, Pichia Pastoris or Cladosporium cell, algal or cyanobacterial cell, a plant cell such as maize or rice cell or mammalian cell such as a mouse or human cell. Preferably, the host cell of the present invention comprises a nucleic acid, wherein the nucleic acid is encoding for the beta 1,6-glucan binding polypeptide as defined herein, or a beta 1,6-glucan binding fragment of the present invention, the fusion protein as defined herein, or the vector of the present invention.

Organisms of the invention may comprise any organism, in particular non-human organism, including preferably bacteria, yeast, fungi, algae or cyanobacteria, plant and animal organisms such as mice or birds. Preferably, the non-human organism comprises a nucleic acid, wherein the nucleic acid is encoding for the beta 1,6-glucan binding polypeptide as defined herein, in particular the polypeptide of the present invention, the polynucleotide of the present invention, or a beta 1,6-glucan binding fragment of the present invention, the fusion protein as defined herein, or the vector of the present invention.

Preferably, the nucleic acid comprised by the organism or host cell is a recombinant nucleic acid. Thus, it is envisaged that the nucleic acid is heterologous with respect to host cell and non-human organism.

Preferably, the nucleic acid comprised by the host cell or non-human organism of the present invention is operably linked to a heterologous expression control sequence.

The present invention also relates to the use of the conjugate of the present invention, the fragment of the present invention, the vector of the present invention for detecting beta 1,6 glucans and/or fungal cells in a sample, or for diagnosing fungal infection in a sample.
In an embodiment, said use is an in vivo use. In an alternative embodiment, said use in an in vitro use.

The term "sample" as used herein refers to samples from any source which are suspected to contain to contain beta 1,6 glucans and/or fungal cells. Preferably, said sample are derived from biological sources. Samples from biological sources (i.e. biological samples) usually comprise a plurality of compounds. Biological samples to be used in the method of the present invention are samples from various organisms. Preferably, the samples are derived from embryophyta (terrestrial plants) or animals. Said samples can be derived from various sources within an organisms and include, for example, tissue samples from plants such as leaf or seed samples, body fluids from animals such as blood, plasma, serum, lymph, or urine, or samples derived, e.g., by biopsy, from cells, tissues or organs. Also encompassed are samples comprising subcellular compartments or organelles, such as the mitochondria, Golgi network or peroxisomes. Samples can also be derived from a subject as specified elsewhere herein. Techniques for obtaining the aforementioned different types of samples are well known in the art. For example, blood samples may be obtained by blood taking while tissue or organ samples are to be obtained, e.g., by biopsy.

In an embodiment, it is envisaged that the sample is body fluid such as urine, in particular blood, serum or plasma.

Further, the conjugate, the fragment, the vector or the host cell or non-human organism of the present invention can be used for a variety of applications including biology, biochemical, biomedical or diagnostic assays such as, for example, fluorescence in situ hybridization (FISH), histochemistry, Enzyme Linked Immunosorbent Assay (ELISA) or microscopy.

Further encompassed by the present invention is a method for the detection of beta 1,6 glucans and/or fungal cells in a sample, said method comprising, providing a sample suspected to comprise beta 1,6 glucans and/or fungal cells, and contacting said sample with the conjugate of the present invention, the polypeptide of the present invention, the fragment of the present invention, the vector of the present invention or the host cell non-human organism of the present invention.

In a preferred embodiment, the sample is contacted with the conjugate of the present invention. The presence (or absence) of beta 1,6 glucans and/or fungal cells can be detected via the detectable label, i.e. by detecting the detectable label.

The term "sample" has been defined above. In an embodiment the sample in accordance with the present invention from embryophyta (terrestrial plants). In another embodiment, the sample is from an animal, in particular from a mammal, preferably a human.

The present invention also relates to a method for diagnosing fungal infection in a subject, said method comprising, providing a sample from a subject suspected to have a fungal infection, contacting said sample with the conjugate of the present invention, the polypeptide of the present invention, or the fragment of the present invention. Thereby, the presence or absence of fungal infection in said subject is diagnosed.

The sample can be any sample from the subject such as a body fluid as set forth above.

The term "subject" as used herein relates to animals, preferably to mammals such as mice, rats, sheep, dogs, cats, horses, monkeys, or cows and, also preferably, to humans. Other animals which may be diagnosed applying the method of the present invention are birds or reptiles. A subject suspected to have a fungal infection as used herein refers to a subject which shows, preferably, symptoms or clinical signs or parameters indicative for a fungal infection However, the term also relates to an apparently healthy subject, i.e. a subject not exhibiting any of the aforementioned symptoms, clinical signs or parameters. Apparently healthy subjects may by investigated by the method of the present invention as a measure of preventive care or for population screening purposes.

The presence (or absence) of beta 1,6 glucans and/or fungal cells can be detected via the detectable label, i.e. by detecting the detectable label.

Moreover, the present invention encompasses a kit comprising the conjugate of the present invention, the polypeptide of the present invention, the polynucleotide of the present invention, the antibody of the present invention, the fragment of the present invention, the vector of the present invention or the host cell or non-human organism of the present invention. Said kit may further comprise means for detecting the detectable label and optionally a instructions for using the kit.

The term "kit" as used herein refers to a collection of the aforementioned components, preferably, provided separately or within a single container.

The present invention further relates to a beta 1,6-glucan binding polypeptide, wherein said polypeptide comprise an amino acid sequence as shown in any one of SEQ ID NO: 2 and 8 to 35, or wherein said polypeptide is a beta 1,6-glucan binding variant of the polypeptide comprising an amino acid sequence as shown in any one of SEQ ID NO: 2 and 8 to 35.

In a preferred embodiment, the beta 1,6-glucan binding polypeptide comprises an amino acid sequence as shown in SEQ ID NO: 2, or is a beta 1,6-glucan binding variant of the polypeptide comprising an amino acid sequence as shown in any one of SEQ ID NO: 2.

The term "variant" is defined elsewhere herein. Preferably, said variant comprises (or has) an amino acid sequence which is at least 50%, at least 60 %, at least 70%, at least 75%, at least 80%, at least 85%, at least 90%, at least 95%, at least 96%, at least 97%, at least 98% or at least 99% identical to the amino acid as shown in SEQ ID NO: 2. Moreover, the variant preferably, comprises conserved domains, conserved cysteins, and/or Motif 1, 2, 3, and 4 as described herein above in detail.

Preferably, the variant of the polypeptide comprising an amino acid sequence as shown in SEQ ID NO: 2 does not have or comprise a sequence as shown in SEQ ID NO: 38, and/or is not encoded by a polynucleotide having or comprising a sequence as shown in SEQ ID NO: 39. However, variants or fragments of the said polypeptide are encompassed by the present invention.

The present invention further relates to a polynucleotide encoding the beta 1,6-glucan binding fragment, or the beta 1,6-glucan binding polypeptide of the present invention, or a fusion protein as defined herein (in connection with the conjugate). Further, the present invention relates to a variant of said polynucleotide. Said variant shall encode a beta 1,6-glucan binding polypeptide. In an embodiment, the polynucleotide (or variant) does not have or comprise a sequence as shown in SEQ ID NO: 39. However, variants or fragments of the said polypeptide are encompassed by the present invention.

The present invention further relates to a complex comprising
i) at least one beta 1,6 glucan, and
ii) the polypeptide of the present invention (or variant thereof), the fragment of the present invention (or variant thereof), or the conjugate of the present invention.

In the complex, the polypeptide, fragment or conjugate shall be bound to the at least beta 1,6 glucan (i.e. one or more beta 1,6 glucan). Preferably, the beta-1,6-glucan is covalently bound to beta 1,3 glucan (or beta 1,2 glucan), in particular in the form of branching polysaccharides.

The complex may be comprised by a sample. A definition for the term sample is given elsewhere herein. E.g. the sample may be a blood, serum or plasma sample. Further, the complex may be present in a cell wall of a fungus. Said cell wall may be present in a the sample. According, the present invention also relates to a sample comprising the complex, in particular wherein the complex is present, i.e. comprised by a cell wall of a fungus. In an embodiment, said cell wall is not a cell wall of Piriformospora indica (strain DSM 11827). Preferably, the fragment or polypeptide as used herein is a recombinant fragment or polypeptide.

Moreover, the present invention relates to a peptide comprising or consisting of one of the following amino acid sequences:
Motif 1: CDYGVRTSCQK (SEQ ID NO: 40)
Motif 2:GHVYQ (SEQ ID NO: 41)
Motif 3: GLHCGDG (SEQ ID NO: 42)
Motif 4: GHVYQVGSDTNVCDYGVRTSCQK (SEQ ID NO: 43)

Preferably, said peptide comprises less than 30 amino acids. More preferably, said peptide comprises less than 20 amino acids.

Further the present invention relates to an antibody which specifically binds to the polypeptide (or variant thereof) or fragment (or variant thereof) of the present invention.

Antibodies as referred to herein include polyclonal and, in particular, monoclonal antibodies, as well as antigen binding fragments thereof, such as Fv, Fab and F(ab)2 fragments.

In a preferred embodiment, the antibody specifically binds to the polypeptide amino acid as shown in SEQ ID NO: 2, or beta 1,6-glucan binding fragment, or to a variant of said polypeptide of said fragment.

In a particularly preferred embodiment, the antibody specifically binds to peptide of the present invention, in particular to an epitope contained in the peptide.

Further, the present invention relates to a conjugate comprising the beta 1,6-glucan binding fragment or beta 1,6-glucan binding polypeptide of the present invention.

The Figures show:
**Figure 1****:** Alignment of the C-terminal domain of FGB1 from Piriformospora indica PIIN 03211 and further putative beta 1,6-glucan binding polypeptides.
**Figure 2****:** List of beta 1,6-glucan binding polypeptides of the present invention (SEQ ID NO: 2 and 8 to 35), conserved domains/ 1,6-glucan binding fragments (SEQ ID NO: 3, 36 and 37), and motifs comprised by 1,6-glucan binding polypeptides or fragments (motifs 1-4: SEQ ID Nos: 4 to 7).

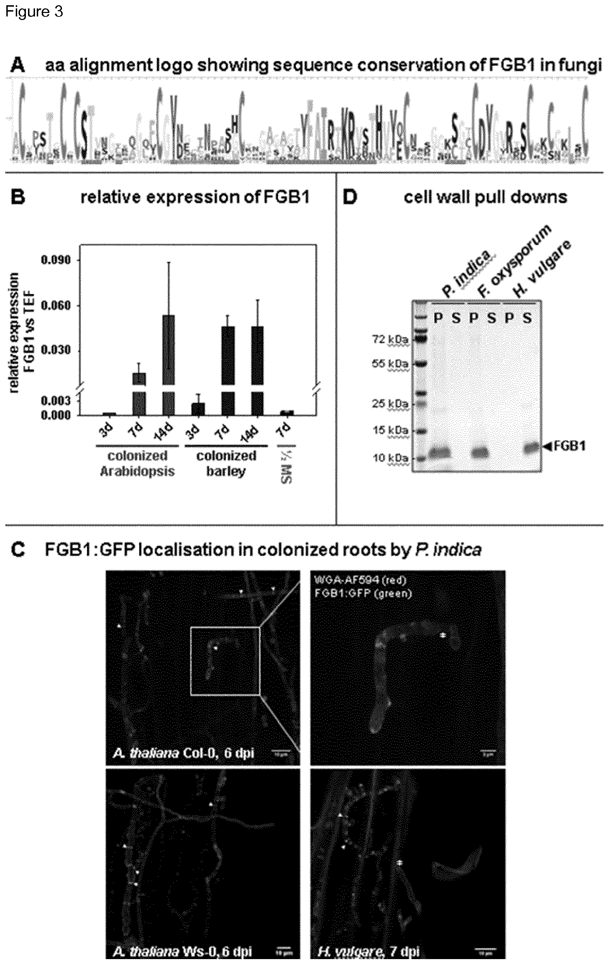

**Figure 3****:** FGB1, a plant responsive small secreted protein from P. indica is capable of binding to fungal cell wall polysaccharides and harbors a novel fungal specific lectin domain. (a) Protein sequence logo of FGB1 (PIIN_3211) obtained after alignment of the mature protein sequence with homologues from different fungi. (b) P. indica FGB1 transcript levels in barley (Hordeum vulgare) and Arabidopsis Col-0 roots grown on ½MS medium inoculated with spores and harvested 3, 7 and 14 dpi and on control ½MS medium (7 dpi) relative to transcription elongation factor (TEF, PIIN_03008). Error bars are standard deviations derived from three biological replicate samples (8 technical replicates each). (c) SDS-PAGE showing the outcome of a pull down experiment probing the binding of FGB1 to protein free cell wall extracts of P. indica, Fusarium oxysporum and barley roots. P: insoluble pellet fraction, S: supernatant. Incubation of FGB1 (20 µM) with the insoluble polysaccharides (10 mg each) was carried out for 1 hr at room temperature in 25 mM sodium-acetate buffer (pH 5.0) containing 500 mM NaCl. (d) FGB1:GFP localizes to the fungal septa (arrowheads) and cell wall in colonized A. thaliana Col-0 and Ws-0 roots (6 dpi) as well as barley roots (7 dpi). The asterisks show the entry point of the hyphae into the root cell. In red the signal from the chitin dye Alexa Fluor 594 labelled wheat germ agglutinin is shown.

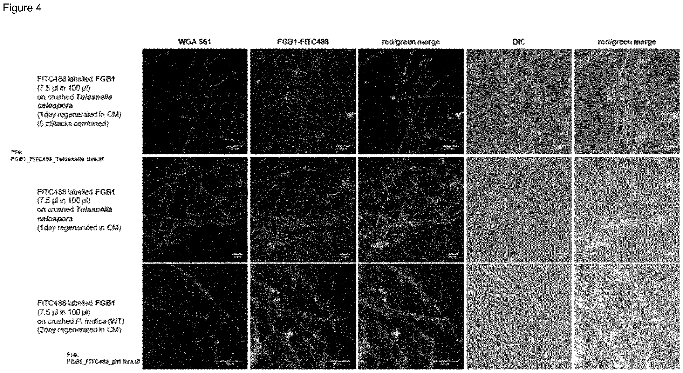

**Figure 4****:** FITC labelled WSC3 (green channel) labels the cell wall of Tulasnella calospora but not P. indica. WGA-AF591 was used as a cell wall counter stain (red channel). In contrast, FITC labelled FGB1 (green channel) labels the cell wall of both T. calospora and P. indica. The labelling on P. indica mycelium with FGB1-FITC488 appeared to be diffuse on the area surrounding the cell wall indicating the labelling of secreted soluble glucan produced by the fungus.

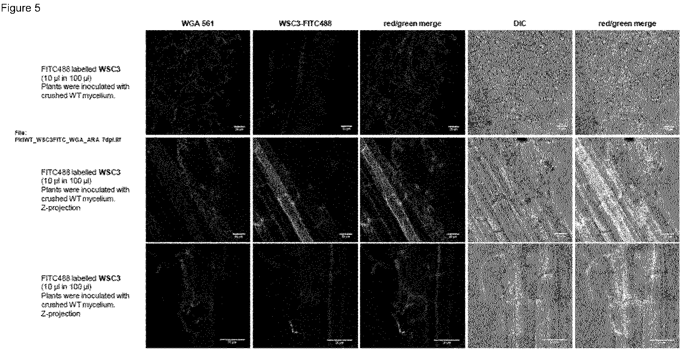

**Figure 5****:** FITC labelled WSC3 (green channel) does not label *P. indica* mycelium and plant cells of inoculated Arabidopsis roots (7dpi).

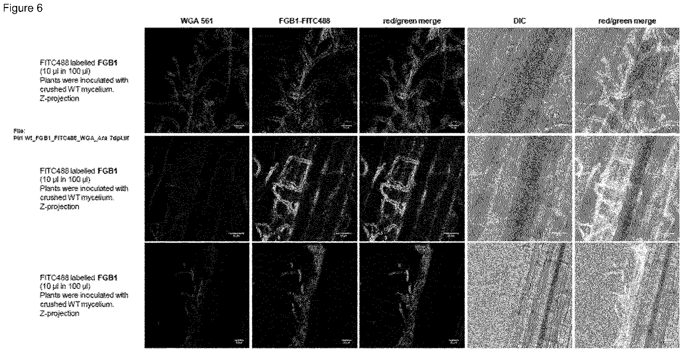

**Figure 6****:** Incubation of P. *indica* inoculated Arabidopsis roots with FITC-488-FGB1 results in a fluorescent signal that surrounds extracellular fungal hyphae like a sheath. This indicates that the protein binds to a soluble glucan polymer produced by the fungus. In addition, one can observe that colonised cells that are filled with green fluorescence signals suggesting that *P. indica* fills these colonized cells with glucan.
**Figure 7****:** A) Protein sequence of FGB1 Piriformospora indica PIIN 03211 (SEQ ID NO: 2) from showing motifs 1 to 4 (SEQ ID NOs: 4 to 7). B) Conserved cysteines at positions 49, 55, 66, 73, 88, 99 and 104 of SEQ ID NO: 2 are highlighted.

### EXAMPLES

The invention will be merely illustrated by the following Examples. The said Examples shall, whatsoever, not be construed in a manner limiting the scope of the invention.

### Example 1: Preparation of P. indica secreted protein fractions, enrichment and characterization of proteins with lectin like properties

To analyze P. indica secreted proteins, the culture filtrate (CF) of one week old cultures were first filtrated through cell strainers with a 40 11 m cut-off before centrifugation for 15 min at 10,000 xg. 5 ml 95% TCA was added to 20 ml cell free CF and incubated overnight at 4"C. Subsequently, 25 ml of 100% acetone 4 was added and the solution was centrifuged for 30 m in at 10,000 xg. Protein pellets were washed with 100% acetone and dried before boiling in Laemmli SDS sample buffer containing 8 M urea and 2 M thiourea.

2 liters of culture filtrate of 7 day old *P. indica* cultures were harvested by filtration through Miracloth (Merckmillipore) and subsequent filtration through 0.22 µm sterile nitrocellulose filters. The obtained cell free CF was pH adjusted with phosphate buffer to pH 7.5 and separated via 40 ml Fractogei-EMD- SO3- (M) (Merck) and EMD TMAE Hicap (M) (Merck) ion exchange material into acidic- and alkaline protein fractions. The obtained protein fractions were dialyzed against 10 mM sodium acetate buffer containing 150 mM NaCl pH 5.0 and 400 111 were incubated with 50 111 sepharose beads for 30 min at room temperature before washing with an excess of binding buffer. Subsequently the sepharose beads were boiled in Laemmli SDS-buffer and bound proteins were analyzed by SDS-PAGE.

### Purification of native FGB1 (PIIN_03211)

To purify native FGB1 the supernatant of 7 day old *P. indica* cultures (2 I) grown in CM-medium was filtrated through Miracloth (Merckmillipore) and subsequently filtrated through 0.22 µm sterile nitrocellulose filters. The obtained cell free culture filtrate was diluted 1:1 with water and supplemented with 1 mM PMSF and 15 ml 1 M sodium acetate buffer pH 5.0 per 4 L to raise the pH of the culture to pH ∼5. The CF was then applied to columns containing EMD TMAE Hicap (M) (Merck) ion exchange material. The flow through of this step was then applied to columns containing Fractogei-EMD-SO³⁻ (M) (Merck) ion exchange material. After protein binding the column was washed with 10 volumes of 10 mMsodium acetate buffer pH 5.0. The crude FGB1 concentrate was obtained by step elution of boundproteins with 1 0 mM sodium acetate buffer pH 5.0 containing 1.5 M NaCl. Final purification was done by applying the SO³⁻ eluate to a spin concentrator with a 30,000 Da cut-off. The flow through of this step was then concentrated using a 5,000 Da cut-off spin concentrator and the concentrated protein was 2x dialyzed (1x 3hr, 1x overnight) against 3 L of water.

### Analysis of FGB1 binding capabilities to cell wall preparations

To assess the binding capability of FGB1 to protein-free cell wall preparations of barley roots (17 days old), *Fusarium oxysporum* (6 day old mycelium) and *P. indica* (6 day old mycelium) the plant and fungal samples were grinded in liquid nitrogen. Subsequently, water was added to the powder and the suspensions were centrifuged for 15 min at 8,000 xg. The resulting pellets were homogenized using an UltraTurax with 8 M urea containing 2% SDS and incubated for 2 hr at 80°C. This was followed by centrifugation for 15 m in at 8,000 xg. The supernatant was removed and the pellets were homogenized with 10 mM sodium phosphate buffer pH 7.5 and centrifuged again. This step was repeated 3-4 times. Subsequently, the pellets were homogenized with PBS containing 0.3 mg/ml Proteinase K and incubated overnight at 37"C. The cell wall polysaccharides were pelleted, homogenized in 2% SDS + BM Urea and incubated at 80°C for 2 hr before centrifugation. The supernatant was removed and the pellets were homogenized in water and centrifuged again (3-4 repeats). The pellets were then homogenized 2x with 100% acetone and pelleted before drying at 80°C. Prior use the obtained polysaccharide pellets were grinded to a fine powder.

For polysaccharide pull downs 10 mg powder was weighted into testtubes and 500 µl water was added. The polysaccharides were then sonicated for 30 s before the addition of 200 µl protein solution to obtain a final buffer conditions of 10 mM sodium acetate pH 5.0 containing 300 mM NaCl and 20 µM FGB1. The mixture was incubated for 1 hr at room temperature before centrifugation for 10 min at 17,000 xg. Supernatant fractions were obtained by removal of 600 µl clear protein solution that was precipitated with 500 µl 40% TCA overnight at 4°C. The supernatant samples were centrifuges for 30 min at 17,000 xg (4°C) and the pellets were washed 2x with 100 % acetone before drying. SDS-PAGE samples were obtained by boiling of for 10 m in at 100 C with 100 µl Laemmli SDS sample buffer containing 8 M urea and 2 M thiourea and 7.5 µl were loaded onto SDS gels.
Pellet fractions were washed 3x with buffer. 100 µl of protein sample buffer was added to the wet pellets and boiled. 15µlof the pellet fractions were loaded onto SDS-gels for analysis.

### Example 2: Database searches and sequence alignments

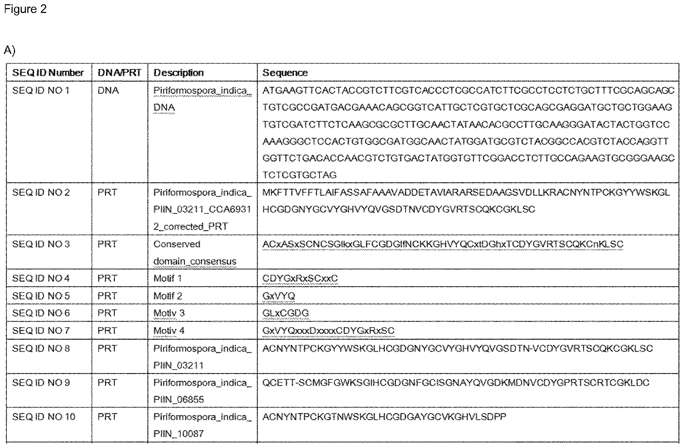

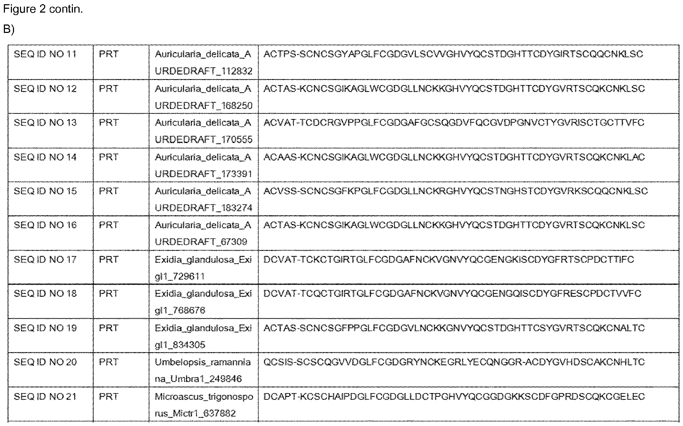

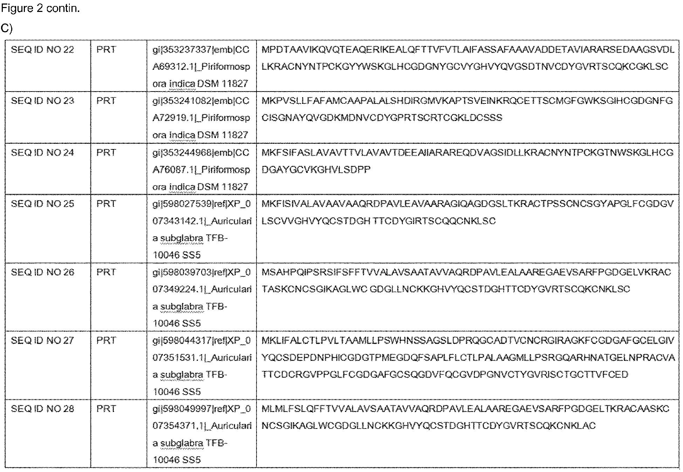

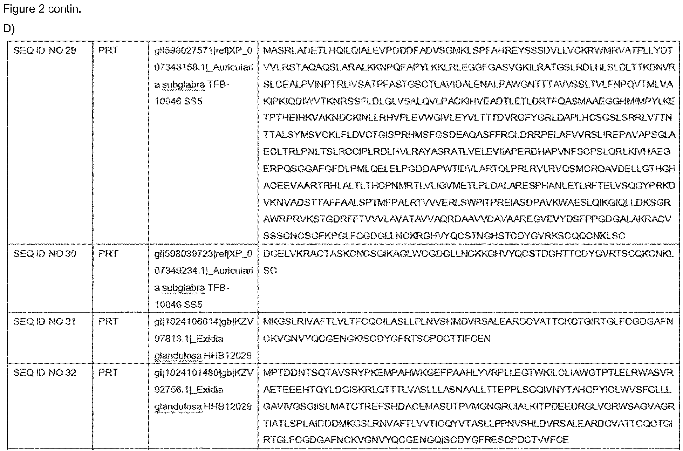

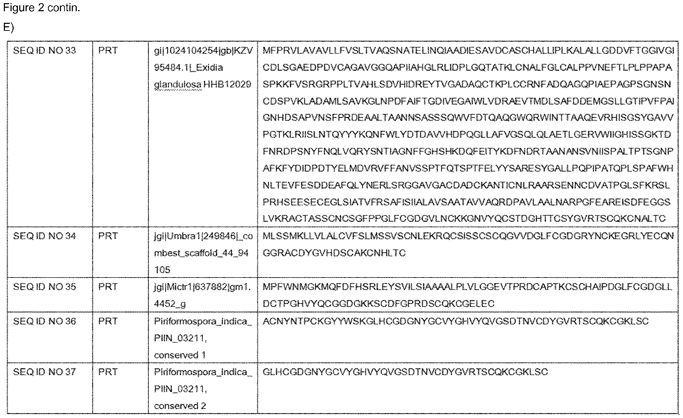

BlastP search with the FGB1 amino acid sequence was performed against Genbank nr database (1.0E-3) and JGI MycoCosm (1.0E-3). Alignment was performed with Mucle using Mega6 and visualized using Maestro 10.3 (Schroedinger Inc.).
Sequence aligments of the FGB1 amino acid sequence and further putative beta 1,6-glucan binding polypeptides are shown in Figures 1 and 2.

### Example 3: Cloning of vectors, expression of an FGB1:GFP fusion protein

### DNA and RNA extraction and cloning procedures

DNA and RNA extractions and real-time quantitative PCR procedures as well as the vector pGoGFP are described in Hilbert et al. 2012. All primers are listed in the table below. The FGB1 sequence containing the signal peptide and lacking the stop codon was amplified from cDNA and cloned into pGoGFP using the C/a/ and Hind /I/ restriction sites. The FGB 1 gene was in frame with a C-terminal GFP sequence in order to produce the FGB1 :GFP fusion protein when transcribed and translated. In order to substitute the GPD-promoter sequence of pGoGFP with that of FGB 1 the GPD promoter was removed using the Apal and C/a/ restriction sites. Part of the FGB1 promoter was amplified using suitable primers and fused to the remaining nucleotide sequence obtained from GenScript (sequence is shown in Appendix). The whole construct was then cloned into pGoGFP using the Apal and ClaI restriction sites.

### Example 4: Analysis and characterization of recombinant FGB1 expression

Sample preparation for comparison of the FGB1 expression level in liquid CM and YNB

### medium

In order to account for the different spore germination rates in CM and YNB medium mycelium a one week old *P. indica* WT culture grown in CM (50 ml) was filtrated and washed with 0.9% NaCl before crushing in 50 ml liquid CM. Subsequently, the mycelium was regenerated for two days. After regeneration the mycelium was filtrated and washed again and 0.5 g was used to inoculate either 25 ml CM or YNB medium (3 replicates each). Cultures were grown for the indicated times at 28°C before mycelium was harvested and RNAcDNA preparation and qPCR were performed.

### Confocal microscopy

Images were recorded using a Leica TCS SP8 confocal microscope. GFP imaging was done with an excitation at 488 nm and detection of the emitted light between 500-540 nm. FM4-64 (Molecular Probes, Karlsruhe, Germany) staining was carried out for 5 min after addition of 1 µl of a 2 mM stock solution dissolved in DMSO per 1 ml of culture and imaging was done using an excitation wavelength of 561 nm and detection between 580-660 nm. For wheat germ agglutinin-Aiexa Fluor 594 (WGA-AF594, Invitrogen) 2 µl of a 1 mg/ml stock solution (dissolved in PBS) was used in a volume of 0.5 ml (excitation at 561 nm detection at 610-650 nm).

### Maize infection assays for U. maydis

Seven day old maize seedlings were infected with the respective *U. maydis* SG200 transformant strains according to Djamei *et al.* (2011) either expressing FGB1:GFP or GFP under the control of the *P. indica* GPD promotor (plasmid pGoGFP). Disease symptoms were scored 12 dpi according to Djamei *et al.* (2011) for three independent isolates each and three biological replicates.

### Isothermal titration calorimetry (ITC)

Isothermal titration experiments were performed to analyze the affinity of FGB1 to different soluble oligo-and polysaccharides using a VP-ITC instrument at 20°C. The instrument was heat-pulse-calibrated and the protein samples were extensively dialyzed against water. Titrant stock solutions were prepared with the same batch of water as used for dialysis. All solutions used were degassed before filling the sample cell and syringe. Compound concentrations are given in the figure legends for the individual experiments. The ITC stirring speed was set to 300 rpm; the feedback gain mode was set to medium. Since the initial injection generally delivers inaccurate data, the first step was omitted from the analysis. The collected data were analyzed using the program "Origin" (MicroCal) and binding isotherms were fitted using the binding models provided by the supplier. Errors correspond to the standard deviation (SD) of the non linear least-squares fit of the data points of the titration curve.

### Circular dichroism (CD) spectroscopy

In order to gain information about the FGB 1 secondary structure in presence and absence of different oligo- and polysaccharides CD-spectra were recorded on a Jasco J700- in degassed water at 20°C. Protein and ligand concentrations are given in the respective figure legends. All spectra were subtracted with the data obtained for the water blank.

### SOS-PAGE and western blotting

SDS-PAGEs were essentially performed according to the manufacturer's instructions (Invitrogen). Gradient 4-12% Bis-Tris NuPage gels were used with NuPage MES-SDS running buffer (Invitrogen). Protein samples were dissolved in Laemmli SDS buffer containing 8 M urea, 2 M thiourea and 2% beta-mercaptoethanol. For western blotting protein from unstained gels were transferred onto nitrocellulose membranes using a semi dry blotting system from Biorad. Anti GFP antibody was obtained from Roche (#11814460001) and used at a 1:2000 dilution. As secondary antibody an anti-mouse antibody purchased from Sigma (A2304) was used at a dilution of 1:2000.

### Thin layer chromatography (TLC), in vitro glucanase activity assay

In order to test whether FGB1 is able to protect beta-glucan from hydrolysis by glucanases an endo-beta-1,3-D-glucanase and an exo- beta-1,3-D-glucanase, from barley and *Trichoderma virens* respectively, were incubated with laminarin in the presence and absence of FGB1. After mixing of the individualcompounds (concentrations are given in the figure legend) the reactions were kept at 37"C (700 rpm shaking) for 15 m in in case of the reaction assays containing the endo-glucanase and 1 hr in case of the exo-glucanase. 4x 1.5 µl assay were spotted per sample on silica-gel 60 F254 plates (Merck, 1.16834). TLC was performed in n-butanol-Isopropanol-water (3:12:4). After the run the plates were dried at RT and developed by spraying with 1-napthol (15 mg of napthol in 12.4 ml ethanol+ 1 ml H20+ 1.6 ml H2SO4 and subsequent incubation at 20°"C (ca. 30s). Barley endo-beta-1,3-D-glucanase glucanase (E-LAM HV) and *Trichoderma virens* exo-131 ,3-o-glucanase (E-EXBGTV) were obtained from Megazyme.

### BCA assay

Laminarin (from 0.6 to 5 g/l = 8.53-M to 1 mM) was incubated with either exo-1 ,3-13-D-glucanase *(Trichoderma virens,* Megazyme® E-EXBGTV) in sodium acetate buffer (100 mM, pH 4.5) or endo-1,3-13-o-glucanase (Barley, Megazyme® E-LAMHV) in sodium acetate buffer (100 mM, pH5.2) (12,5 or 25 U/1) at 40°C and 900 rpm (EppendorfThermomixer) and FGB1 (5.83 to 42.53 ∼M) was added. Samples were taken at different time intervals and the reaction was stopped by heating the samples at 95°C for 5 minutes. The released glucose was quantified with the bicinchoninic acid (BCA)-assay via the optical density at 540 nm with a 96well plate reader (TECAN Sunrise) using glucose as a standard. The BCA assay was performed according to McFeeters et al. (1980). In brief, this assay relies on the proportional reduction of Cu2+ (from CuSO4) to Cu+ (biuret reaction) by the reducing sugars in solution. Calorimetric quantification was performed subsequently to the chelation Cu+ by bicinchoninic acid sodium salt (1:2 stoichiometry). The formed complex strongly absorbs light at a wavelength of 540-560 nm in an alkaline environment.

### Determination of the glucose to N-acetyl glucosamine ratio of fungal cell walls after total hydrolysis

Deuterated hydrochloric acid (30% DCI in D₂0) was used to catalyze the acidic hydrolysis of polymers from the non-soluble fraction of the cell wall to their monomers (Einbu et al. 2007). The ratio of glucose and N-acetyl glucosamine was determined after hydrolysis by the distribution via 1H-NMR-spectroscopy (Bruker DMX 600 FT-NMR-spectrometer; 1H: 600 MHz at 295 K). The NMR-spectra were calibrated by setting the water signal to 9.10 ppm. The amount of N-acetylglucosamine was calculated using the integrated signal of the acetylic group at 2.26 ppm. The amount of glucose was calculated from the sum of all anomeric protons minus that of the N-acetylglucosamine. The 1H-NMR exhibited three anomeric protons at 5.03 ppm, at 4.50 ppm (alpha-D-glucose, beta-3-D-glucose, N-acetylglucosamine) and a third signal at 5.98 ppm corresponding to 1,6-anhydro-beta-D-glucose. Under strong acidic conditions glucose undergoes a dehydration reaction to 1,6-anhydro-beta-D-glucose. The different samples had a dry mass weight between 10 to 25 mg and were dissolved in 750µl DCI.

### Apoplastic fluid collection from P. indica colonized barley roots

Barley seeds *(Hordeum vulgare* L. cv Golden Promise) were surface sterilized for 1 min under light shaking in 70% ethanol followed by 1 hour in 12% sodium hypochlorite. Subsequently, seeds were washed repeatedly for 1 hour with sterile distilled water. After germination for three days at 22'C in complete darkness on wet filter paper, germinated seeds were transferred into jars with 1/10 PNM (Basiewicz et al. 2012) under sterile conditions and inoculated with 3 ml of chlamydospores (concentration 500,000 spores/ml) of *P .indica* GoGFP strain (Hilbert et al. 2012) and cultivated in a growth chamber with a day: night cycle of 16 h: 8 h (light intensity, 108 ∼mol m2/s) and temperature of 22°C : 18°C. For apoplastic fluid (APF) extraction three time points reflecting different interaction stages have been chosen: Early and late biotrophic phase (5 and 10 dpi) as well as cell death associated phase (14 dpi). After respective growth time, roots were thoroughly washed and cut into pieces of 2 cm length (2nd to 4th cm of the root). Samples were vacuum infiltrated (Vacuumbrand, CVC 3000, VWR) with deionized water 5x15 min at 250 mbar with 1 m in atmospheric pressure break. Bundled infiltrated roots were centrifuged in 5 ml syringe barrels at 2000 rpm for 15 m in at 4°C. Pooled APF was stored at -20'C. In order to exclude cytoplasmic contamination apoplastic fluid was tested in immunoblots with GFP antibody. For higher peptide mass accuracy APF was deglycosylated with Protein Deglycosylation Mix (P6039S, New England BioLabs, Ipswich, USA) under denaturing conditions. For peptide mass identification samples were digested with trypsin and subjected to LC-MS/MS mass spectrometry. Proteins matched to peptides were analyzed for the presence of signal peptides and exclusiveness in the three specific time points of APF extraction.

### Sequences comprised by the sequence listing:

| **SEQ ID Number** | **DNA/PRT** | **Description** |
|---|---|---|
| SEQ ID NO 1 | DNA | Piriformospora_indica_DNA |
| SEQ ID NO 2 | PRT | Piriformospora_indica_PIIN_03211_CCA69312_corrected_PRT |
| SEQ ID NO 3 | PRT | Conserved domain_consensus |
| SEQ ID NO 4 | PRT | Motif 1 |
| SEQ ID NO 5 | PRT | Motif 2 |
| SEQ ID NO 6 | PRT | Motiv 3 |
| SEQ ID NO 7 | PRT | Motiv 4 |
| SEQ ID NO 8 | PRT | Piriformospora_indica_PIIN_03211 |
| SEQ ID NO 9 | PRT | Piriformospora_indica_PIIN_06855 |
| SEQ ID NO 10 | PRT | Piriformospora_indica_PIIN_10087 |
| SEQ ID NO 11 | PRT | Auricularia_delicata_AURDEDRAFT_112832 |
| SEQ ID NO 12 | PRT | Auricularia_delicata_AURDEDRAFT_168250 |
| SEQ ID NO 13 | PRT | Auricularia_delicata_AURDEDRAFT_170555 |
| SEQ ID NO 14 | PRT | Auricularia_delicata_AURDEDRAFT_173391 |
| SEQ ID NO 15 | PRT | Auricularia_delicata_AURDEDRAFT_183274 |
| SEQ ID NO 16 | PRT | Auricularia_delicata_AURDEDRAFT_67309 |
| SEQ ID NO 17 | PRT | Exidia_glandulosa_Exigl1_729611 |
| SEQ ID NO 18 | PRT | Exidia_glandulosa_Exigl1_768676 |
| SEQ ID NO 19 | PRT | Exidia_glandulosa_Exigl1_834305 |
| SEQ ID NO 20 | PRT | Umbelopsis_ramanniana_Umbra1_249846 |
| SEQ ID NO 21 | PRT | Microascus_trigonosporus_Mictr1_637882 |
| SEQ ID NO 22 | PRT | gi\|353237337\|emb\|CCA69312.1\|_Piriformospora indica DSM 11827 |
| SEQ ID NO 23 | PRT | gi\|353241082\|emb\|CCA72919.1\|_Piriformospora indica DSM 11827 |
| SEQ ID NO 24 | PRT | gi\|353244968\|emb\|CCA76087.1\|_Piriformospora indica DSM 11827 |
| SEQ ID NO 25 | PRT | gi\|598027539\|ref\|XP_007343142.1\|_Auricularia subglabra TFB-10046 SS5 |
| SEQ ID NO 26 | PRT | gi\|598039703\|ref\|XP_007349224.1\|_Auricularia subglabra TFB-10046 SS5 |
| SEQ ID NO 27 | PRT | gi\|598044317\|ref\|XP_007351531.1\|-Auricularia subglabra TFB-10046 SS5 |
| SEQ ID NO 28 | PRT | gi\|598049997\|ref\|XP_007354371.1\|_Auricularia subglabra TFB-10046 SS5 |
| SEQ ID NO 29 | PRT | gi\|598027571\|ref\|XP_007343158.1\|_Auricularia subglabra TFB-10046 SS5 |
| SEQ ID NO 30 | PRT | gi\|598039723\|ref\|XP_007349234.1\|_Auricularia subglabra TFB-10046 SS5 |
| SEQ ID NO 31 | PRT | gi\|1024106614\|gb\|KZV97813.1\|_Exidia glandulosa HHB12029 |
| SEQ ID NO 32 | PRT | gi\|1024101480\|gb\|KZV92756.1\|_Exidia glandulosa HHB12029 |
| SEQ ID NO 33 | PRT | gi\|1024104254\|gb\|KZV95484.1\|_Exidia glandulosa HHB12029 |
| SEQ ID NO 34 | PRT | jgi\|Umbra1\|249846\|_combest_scaffold_44_94105 |
| SEQ ID NO 35 | PRT | jgi\|Mictr1\|637882\|gm1.4452_g |

### Full citations of the references cited throughout the specification

Tamura K, Stecher G, Peterson D, Filipski A, Kumar S. MEGA6: Molecular Evolutionary Genetics Analysis version 6.0. Molecular biology and evolution 30, 2725-2729 (2013).
Hilbert M, Voll LM, DingY, Hofmann J, Sharma M, Zuccaro A. Indole derivative production by the root endophyte Piriformospora indica is not required for growth promotion but for biotrophic colonization of barley roots. The New phyfologist 196, 520-534 (2012).
Djamei A, et al. Metabolic priming by a secreted fungal effector. Nature 478, 395-398 (2011).
McFeeters RF. A manual method for reducing sugar determinations with 2,2'-bicinchoninate reagent. Anal Biochem 103,302-306 (1980).
Einbu A, Grasdalen H, Varum KM. Kinetics of hydrolysis of chitin/chitosan oligomers in concentrated hydrochloric acid. Carbohydrate research 342, 1055-1062 (2007).

## Claims

1. A conjugate comprising i) a beta 1,6-glucan binding polypeptide, or a beta 1,6-glucan binding fragment of said polypeptide, and ii) a detectable label.

2. The conjugate of claim 1, wherein the detectable label is covalently bound to said beta 1,6-glucan binding polypeptide, or said beta 1,6-glucan binding fragment thereof, or the detectable label is non-covalently bound to said a beta 1,6-glucan binding polypeptide, or a beta 1,6-glucan binding fragment thereof.

3. The conjugate of claims 1 and 2, wherein the detectable label is a visual label.

4. The conjugate of any one of claims 1 to 3, wherein the detectable label is selected from the group consisting of expressed fluorescent proteins, fluorescent peptide or protein labels, fluorescent dyes, inorganic nanocrystals or nanoparticles with unique optical and chemical properties, chromogens, luminescent or chemiluminescent compounds, radioactive compounds, a combination of an enzyme and its substrate, amino acids, peptides or antibodies linked to signal producing substances, a liposome or vesicle containing signal producing substances and electroactive species.

5. The conjugate of any one of claims 1 to 4, wherein the detectable label is a fluorescent label selected from the group consisting of Green Fluorescent Protein (GFP), Cyan Fluorescent Protein (CFP), Red Fluorescent Protein (RFP), Allophycocyanin (APC), R-Phycoerythrin (R-PE), and a fluorescent dye.

6. The conjugate of any one of claims 1 to 5, wherein the detectable label is a peptide or polypeptide, and wherein said conjugate is a fusion polypeptide comprising the beta 1,6-glucan binding polypeptide, or the beta 1,6-glucan binding fragment thereof, and said detectable label.

7. The conjugate of any one claim 1 to 6, wherein the beta 1,6-glucan binding polypeptide comprises an amino acid sequence as shown in any one of SEQ ID NO: 2 and 8 to 35, or is a beta 1,6-glucan binding variant thereof.

8. A fragment of a beta 1,6-glucan binding polypeptide or variant thereof, wherein said beta 1,6-glucan binding polypeptide has an amino acid sequence as shown in any one of SEQ ID NOs: 2 and 8 to 35, and wherein the fragment specifically binds to beta 1,6 glucans.

9. The fragment of claim 8, wherein said fragment has a length of at least 20 amino acids.

10. A vector comprising a nucleic acid encoding for the beta 1,6-glucan binding polypeptide as defined in claims 1, 2 or 7" or a beta 1,6-glucan binding fragment thereof as defined in claims 1 and 2, the fusion protein as defined in claim 6, or the fragment of claims 8 and 9.

11. The vector of claim 10, wherein said nucleic acid is operably linked to a heterologous expression control sequence.

12. A host cell or non-human organism comprising a nucleic acid, wherein the nucleic acid is encoding for the beta 1,6-glucan binding polypeptide as defined in claim 1 and 7, or a beta 1,6-glucan binding fragment thereof as defined in claims 1 and 2, the fusion protein as defined in claim 6, the fragment of claims 8 and 9, or the vector of claims 10 and 11.

13. Use of the conjugate as defined in claims 1 to 7, the fragment as defined in claims 8 and 9, the polypeptide as defined in claim 7, the vector as defined in claims 10 and 11 for detecting beta 1,6 glucans and/or fungal cells in a sample, or for diagnosing fungal infection.

14. A method for the detection of beta 1,6 glucans and/or fungal cells in a sample, said method comprising, providing a sample suspected to comprise beta 1,6 glucans and/or fungal cells, and contacting said sample with the conjugate according to any one of claims 1 to 7, the polypeptide as defined in claims 7, the fragment of claim 8 or 9, the vector of claims 10 and 11 or the host cell non-human organism of claim 12.

15. A method for diagnosing fungal infection in a subject, said method comprising, providing a sample from a subject suspected to have a fungal infection, contacting said sample with the conjugate according to any one of claims 1 to 7, the polypeptide as defined in claim 7, or the fragment according to any one of claims 8 and 9, thereby diagnosing the presence or absence of fungal infection in said subject.

16. A kit comprising the conjugate according to any one of claims 1 to 7, the fragment according to any one of claims 8 to 9, the polypeptide as defined in claim 7, the vector according to any one of claims 10 to 11 or the host cell or non-human organism according to claim 12.


SEQUENCE LISTING

<110> Universitaet zu Koeln

<120> MEANS AND METHODS FOR DETECTING BETA-1,6-GLUCAN

<130> P14032EP

<160> 43

<170> BiSSAP 1.3.6

<210> 1
<211> 315
<212> DNA
<213> Serendipita indica


<220>
<223> Piriformospora_indica_PIIN_03211_coding_sequence

<400> 1
atgaagttca ctaccgtctt cgtcaccctc gccatcttcg cctcctctgc tttcgcagca      60

gctgtcgccg atgacgaaac agcggtcatt gctcgtgctc gcagcgagga tgctgctgga     120

agtgtcgatc ttctcaagcg cgcttgcaac tataacacgc cttgcaaggg atactactgg     180

tccaaagggc tccactgtgg cgatggcaac tatggatgcg tctacggcca cgtctaccag     240

gttggttctg acaccaacgt ctgtgactat ggtgttcgga cctcttgcca gaagtgcggg     300

aagctctcgt gctag                                                      315


<210> 2
<211> 104
<212> PRT
<213> Serendipita indica


<220>
<223> Piriformospora_indica_PIIN_03211_CCA69312_corrected_PRT

<400> 2
Met Lys Phe Thr Thr Val Phe Phe Thr Leu Ala Ile Phe Ala Ser Ser
1               5                   10                  15
Ala Phe Ala Ala Ala Val Ala Asp Asp Glu Thr Ala Val Ile Ala Arg
            20                  25                  30
Ala Arg Ser Glu Asp Ala Ala Gly Ser Val Asp Leu Leu Lys Arg Ala
        35                  40                  45
Cys Asn Tyr Asn Thr Pro Cys Lys Gly Tyr Tyr Trp Ser Lys Gly Leu
    50                  55                  60
His Cys Gly Asp Gly Asn Tyr Gly Cys Val Tyr Gly His Val Tyr Gln
65                  70                  75                  80
Val Gly Ser Asp Thr Asn Val Cys Asp Tyr Gly Val Arg Thr Ser Cys
                85                  90                  95
Gln Lys Cys Gly Lys Leu Ser Cys
            100

<210> 3
<211> 58
<212> PRT

<213> artificial sequences

<220>
<223> Conserved domain_consensus sequence

<400> 3
Ala Cys Xaa Ala Ser Xaa Ser Cys Asn Cys Ser Gly Ile Lys Xaa Gly
1               5                   10                  15
Leu Phe Cys Gly Asp Gly Leu Phe Asn Cys Lys Lys Gly His Val Tyr
            20                  25                  30
Gln Cys Xaa Thr Asp Gly His Xaa Thr Cys Asp Tyr Gly Val Arg Thr
        35                  40                  45
Ser Cys Gln Lys Cys Asn Lys Leu Ser Cys
    50                  55

<210> 4
<211> 12
<212> PRT
<213> artificial sequences

<220>
<223> Motif 1

<400> 4
Cys Asp Tyr Gly Xaa Arg Xaa Ser Cys Xaa Xaa Cys
1               5                   10

<210> 5
<211> 5
<212> PRT
<213> artificial sequences

<220>
<223> Motif 2

<400> 5
Gly Xaa Val Tyr Gln
1               5

<210> 6
<211> 7
<212> PRT
<213> artificial sequences

<220>
<223> Motif 3

<400> 6
Gly Leu Xaa Cys Gly Asp Gly
1               5

<210> 7
<211> 22
<212> PRT
<213> artificial sequences

<220>
<223> Motif 4

<400> 7
Gly Xaa Val Tyr Gln Xaa Xaa Xaa Asp Xaa Xaa Xaa Xaa Cys Asp Tyr
1               5                   10                  15
Gly Xaa Arg Xaa Ser Cys
            20

<210> 8
<211> 57
<212> PRT
<213> Serendipita indica

<220>
<223> Piriformospora_indica_PIIN_03211

<400> 8
Ala Cys Asn Tyr Asn Thr Pro Cys Lys Gly Tyr Tyr Trp Ser Lys Gly
1               5                   10                  15
Leu His Cys Gly Asp Gly Asn Tyr Gly Cys Val Tyr Gly His Val Tyr
            20                  25                  30
Gln Val Gly Ser Asp Thr Asn Val Cys Asp Tyr Gly Val Arg Thr Ser
        35                  40                  45
Cys Gln Lys Cys Gly Lys Leu Ser Cys
    50                  55

<210> 9
<211> 57
<212> PRT
<213> Serendipita indica

<220>
<223> Piriformospora_indica_PIIN_06855

<400> 9
Gln Cys Glu Thr Thr Ser Cys Met Gly Phe Gly Trp Lys Ser Gly Ile
1               5                   10                  15
His Cys Gly Asp Gly Asn Phe Gly Cys Ile Ser Gly Asn Ala Tyr Gln
            20                  25                  30
Val Gly Asp Lys Met Asp Asn Val Cys Asp Tyr Gly Pro Arg Thr Ser
        35                  40                  45
Cys Arg Thr Cys Gly Lys Leu Asp Cys
    50                  55

<210> 10
<211> 36
<212> PRT
<213> Serendipita indica

<220>
<223> Piriformospora_indica_PIIN_10087

<400> 10
Ala Cys Asn Tyr Asn Thr Pro Cys Lys Gly Thr Asn Trp Ser Lys Gly
1               5                   10                  15
Leu His Cys Gly Asp Gly Ala Tyr Gly Cys Val Lys Gly His Val Leu
            20                  25                  30
Ser Asp Pro Pro
        35

<210> 11

<211> 57
<212> PRT
<213> Auricularia delicata

<220>
<223> Auricularia_delicata_AURDEDRAFT_112832

<400> 11
Ala Cys Thr Pro Ser Ser Cys Asn Cys Ser Gly Tyr Ala Pro Gly Leu
1               5                   10                  15
Phe Cys Gly Asp Gly Val Leu Ser Cys Val Val Gly His Val Tyr Gln
            20                  25                  30
Cys Ser Thr Asp Gly His Thr Thr Cys Asp Tyr Gly Ile Arg Thr Ser
        35                  40                  45
Cys Gln Gln Cys Asn Lys Leu Ser Cys
    50                  55

<210> 12
<211> 57
<212> PRT
<213> Auricularia delicata

<220>
<223> Auricularia_delicata_AURDEDRAFT_168250

<400> 12
Ala Cys Thr Ala Ser Lys Cys Asn Cys Ser Gly Ile Lys Ala Gly Leu
1               5                   10                  15
Trp Cys Gly Asp Gly Leu Leu Asn Cys Lys Lys Gly His Val Tyr Gln
            20                  25                  30
Cys Ser Thr Asp Gly His Thr Thr Cys Asp Tyr Gly Val Arg Thr Ser
        35                  40                  45
Cys Gln Lys Cys Asn Lys Leu Ser Cys
    50                  55

<210> 13
<211> 57
<212> PRT
<213> Auricularia delicata

<220>
<223> Auricularia_delicata_AURDEDRAFT_168250

<400> 13
Ala Cys Thr Ala Ser Lys Cys Asn Cys Ser Gly Ile Lys Ala Gly Leu
1               5                   10                  15
Trp Cys Gly Asp Gly Leu Leu Asn Cys Lys Lys Gly His Val Tyr Gln
            20                  25                  30
Cys Ser Thr Asp Gly His Thr Thr Cys Asp Tyr Gly Val Arg Thr Ser
        35                  40                  45
Cys Gln Lys Cys Asn Lys Leu Ser Cys
    50                  55

<210> 14
<211> 57
<212> PRT
<213> Auricularia delicata

<220>

<223> Auricularia_delicata_AURDEDRAFT_173391

<400> 14
Ala Cys Ala Ala Ser Lys Cys Asn Cys Ser Gly Ile Lys Ala Gly Leu
1               5                   10                  15
Trp Cys Gly Asp Gly Leu Leu Asn Cys Lys Lys Gly His Val Tyr Gln
            20                  25                  30
Cys Ser Thr Asp Gly His Thr Thr Cys Asp Tyr Gly Val Arg Thr Ser
        35                  40                  45
Cys Gln Lys Cys Asn Lys Leu Ala Cys
    50                  55

<210> 15
<211> 57
<212> PRT
<213> Auricularia delicata


<220>
<223> Auricularia_delicata_AURDEDRAFT_183274

<400> 15
Ala Cys Val Ser Ser Ser Cys Asn Cys Ser Gly Phe Lys Pro Gly Leu
1               5                   10                  15
Phe Cys Gly Asp Gly Leu Leu Asn Cys Lys Arg Gly His Val Tyr Gln
            20                  25                  30
Cys Ser Thr Asn Gly His Ser Thr Cys Asp Tyr Gly Val Arg Lys Ser
        35                  40                  45
Cys Gln Gln Cys Asn Lys Leu Ser Cys
    50                  55

<210> 16
<211> 57
<212> PRT
<213> Auricularia delicata


<220>
<223> Auricularia_delicata_AURDEDRAFT_67309

<400> 16
Ala Cys Thr Ala Ser Lys Cys Asn Cys Ser Gly Ile Lys Ala Gly Leu
1               5                   10                  15
Trp Cys Gly Asp Gly Leu Leu Asn Cys Lys Lys Gly His Val Tyr Gln
            20                  25                  30
Cys Ser Thr Asp Gly His Thr Thr Cys Asp Tyr Gly Val Arg Thr Ser
        35                  40                  45
Cys Gln Lys Cys Asn Lys Leu Ser Cys
    50                  55

<210> 17
<211> 57
<212> PRT
<213> Exidia glandulosa


<220>
<223> Exidia_glandulosa_Exigl1_729611

<400> 17
Asp Cys Val Ala Thr Thr Cys Lys Cys Thr Gly Ile Arg Thr Gly Leu
1               5                   10                  15
Phe Cys Gly Asp Gly Ala Phe Asn Cys Lys Val Gly Asn Val Tyr Gln

                20                  25                  30
Cys Gly Glu Asn Gly Lys Ile Ser Cys Asp Tyr Gly Phe Arg Thr Ser
        35                  40                  45
Cys Pro Asp Cys Thr Thr Ile Phe Cys
    50                  55

<210> 18
<211> 57
<212> PRT
<213> Exidia glandulosa

<220>
<223> Exidia_glandulosa_Exigl1_834305

<400> 18
Ala Cys Thr Ala Ser Ser Cys Asn Cys Ser Gly Phe Pro Pro Gly Leu
1               5                   10                  15
Phe Cys Gly Asp Gly Val Leu Asn Cys Lys Lys Gly Asn Val Tyr Gln
            20                  25                  30
Cys Ser Thr Asp Gly His Thr Thr Cys Ser Tyr Gly Val Arg Thr Ser
        35                  40                  45
Cys Gln Lys Cys Asn Ala Leu Thr Cys
    50                  55

<210> 19
<211> 57
<212> PRT
<213> Exidia glandulosa

<220>
<223> Exidia_glandulosa_Exigl1_834305

<400> 19
Ala Cys Thr Ala Ser Ser Cys Asn Cys Ser Gly Phe Pro Pro Gly Leu
1               5                   10                  15
Phe Cys Gly Asp Gly Val Leu Asn Cys Lys Lys Gly Asn Val Tyr Gln
            20                  25                  30
Cys Ser Thr Asp Gly His Thr Thr Cys Ser Tyr Gly Val Arg Thr Ser
        35                  40                  45
Cys Gln Lys Cys Asn Ala Leu Thr Cys
    50                  55

<210> 20
<211> 56
<212> PRT
<213> Umbelopsis ramanniana

<220>
<223> Umbelopsis_ramanniana_Umbra1_249846

<400> 20
Gln Cys Ser Ile Ser Ser Cys Ser Cys Gln Gly Val Val Asp Gly Leu
1               5                   10                  15
Phe Cys Gly Asp Gly Arg Tyr Asn Cys Lys Glu Gly Arg Leu Tyr Glu
            20                  25                  30
Cys Gln Asn Gly Gly Arg Ala Cys Asp Tyr Gly Val His Asp Ser Cys
        35                  40                  45
Ala Lys Cys Asn His Leu Thr Cys
    50                  55

<210> 21
<211> 57
<212> PRT
<213> Microascus trigonosporus

<220>
<223> Microascus_trigonosporus_Mictr1_637882

<400> 21
Asp Cys Ala Pro Thr Lys Cys Ser Cys His Ala Ile Pro Asp Gly Leu
1               5                   10                  15
Phe Cys Gly Asp Gly Leu Leu Asp Cys Thr Pro Gly His Val Tyr Gln
            20                  25                  30
Cys Gly Gly Asp Gly Lys Lys Ser Cys Asp Phe Gly Pro Arg Asp Ser
        35                  40                  45
Cys Gln Lys Cys Gly Glu Leu Glu Cys
    50                  55

<210> 22
<211> 126
<212> PRT
<213> Serendipita indica

<220>
<223> gi|353237337|emb|CCA69312.1|_Piriformospora indica DSM 118 27

<400> 22
Met Pro Asp Thr Ala Ala Val Ile Lys Gln Val Gln Thr Glu Ala Gln
1               5                   10                  15
Glu Arg Ile Lys Glu Ala Leu Gln Phe Thr Thr Val Phe Val Thr Leu
            20                  25                  30
Ala Ile Phe Ala Ser Ser Ala Phe Ala Ala Ala Val Ala Asp Asp Glu
        35                  40                  45
Thr Ala Val Ile Ala Arg Ala Arg Ser Glu Asp Ala Ala Gly Ser Val
    50                  55                  60
Asp Leu Leu Lys Arg Ala Cys Asn Tyr Asn Thr Pro Cys Lys Gly Tyr
65                  70                  75                  80
Tyr Trp Ser Lys Gly Leu His Cys Gly Asp Gly Asn Tyr Gly Cys Val
                85                  90                  95
Tyr Gly His Val Tyr Gln Val Gly Ser Asp Thr Asn Val Cys Asp Tyr
            100                 105                 110
Gly Val Arg Thr Ser Cys Gln Lys Cys Gly Lys Leu Ser Cys
        115                 120                 125

<210> 23
<211> 99
<212> PRT
<213> Serendipita indica

<220>
<223> gi|353241082|emb|CCA72919.1|_Piriformospora indica DSM 118 27

<400> 23
Met Lys Pro Val Ser Leu Leu Phe Ala Phe Ala Met Cys Ala Ala Pro
1               5                   10                  15
Ala Leu Ala Leu Ser His Asp Ile Arg Gly Met Val Lys Ala Pro Thr
            20                  25                  30
Ser Val Glu Ile Asn Lys Arg Gln Cys Glu Thr Thr Ser Cys Met Gly
        35                  40                  45
Phe Gly Trp Lys Ser Gly Ile His Cys Gly Asp Gly Asn Phe Gly Cys

    50                  55                  60
Ile Ser Gly Asn Ala Tyr Gln Val Gly Asp Lys Met Asp Asn Val Cys
65                  70                  75                  80
Asp Tyr Gly Pro Arg Thr Ser Cys Arg Thr Cys Gly Lys Leu Asp Cys
                85                  90                  95
Ser Ser Ser


<210> 24
<211> 81
<212> PRT
<213> Serendipita indica


<220>
<223> gi|353244968|emb|CCA76087.1|_Piriformospora indica DSM 118 27

<400> 24
Met Lys Phe Ser Ile Phe Ala Ser Leu Ala Val Ala Val Thr Thr Val
1               5                   10                  15
Leu Ala Val Ala Val Thr Asp Glu Glu Ala Ile Ile Ala Arg Ala Arg
            20                  25                  30
Glu Gln Asp Val Ala Gly Ser Ile Asp Leu Leu Lys Arg Ala Cys Asn
        35                  40                  45
Tyr Asn Thr Pro Cys Lys Gly Thr Asn Trp Ser Lys Gly Leu His Cys
    50                  55                  60
Gly Asp Gly Ala Tyr Gly Cys Val Lys Gly His Val Leu Ser Asp Pro
65                  70                  75                  80
Pro


<210> 25
<211> 99
<212> PRT
<213> Auricularia


<220>
<223> gi|598027539|ref|XP_007343142.1|_Auricularia subglabra TFB -10046
      SS5

<400> 25
Met Lys Phe Ile Ser Ile Val Ala Leu Ala Val Ala Ala Val Ala Ala
1               5                   10                  15
Gln Arg Asp Pro Ala Val Leu Glu Ala Val Ala Ala Arg Ala Gly Ile
            20                  25                  30
Gln Ala Gly Asp Gly Ser Leu Thr Lys Arg Ala Cys Thr Pro Ser Ser
        35                  40                  45
Cys Asn Cys Ser Gly Tyr Ala Pro Gly Leu Phe Cys Gly Asp Gly Val
    50                  55                  60
Leu Ser Cys Val Val Gly His Val Tyr Gln Cys Ser Thr Asp Gly His
65                  70                  75                  80
Thr Thr Cys Asp Tyr Gly Ile Arg Thr Ser Cys Gln Gln Cys Asn Lys
                85                  90                  95
Leu Ser Cys


<210> 26
<211> 119
<212> PRT
<213> Auricularia

<220>
<223> gi|598039703|ref|XP_007349224.1|_Auricularia subglabra TFB -10046
      SS5

<400> 26
Met Ser Ala His Pro Gln Ile Pro Ser Arg Ser Ile Phe Ser Phe Phe
1               5                   10                  15
Thr Val Val Ala Leu Ala Val Ser Ala Ala Thr Ala Val Val Ala Gln
            20                  25                  30
Arg Asp Pro Ala Val Leu Glu Ala Leu Ala Ala Arg Glu Gly Ala Glu
        35                  40                  45
Val Ser Ala Arg Phe Pro Gly Asp Gly Glu Leu Val Lys Arg Ala Cys
    50                  55                  60
Thr Ala Ser Lys Cys Asn Cys Ser Gly Ile Lys Ala Gly Leu Trp Cys
65                  70                  75                  80
Gly Asp Gly Leu Leu Asn Cys Lys Lys Gly His Val Tyr Gln Cys Ser
                85                  90                  95
Thr Asp Gly His Thr Thr Cys Asp Tyr Gly Val Arg Thr Ser Cys Gln
            100                 105                 110
Lys Cys Asn Lys Leu Ser Cys
        115

<210> 27
<211> 182
<212> PRT
<213> Auricularia


<220>
<223> gi|598044317|ref|XP_007351531.1|_Auricularia subglabra TFB -10046
      SS5

<400> 27
Met Lys Leu Ile Phe Ala Leu Cys Thr Leu Pro Val Leu Thr Ala Ala
1               5                   10                  15
Met Leu Leu Pro Ser Trp His Asn Ser Ser Ala Gly Ser Leu Asp Pro
            20                  25                  30
Arg Gln Gly Cys Ala Asp Thr Val Cys Asn Cys Arg Gly Ile Arg Ala
        35                  40                  45
Gly Lys Phe Cys Gly Asp Gly Ala Phe Gly Cys Glu Leu Gly Ile Val
    50                  55                  60
Tyr Gln Cys Ser Asp Glu Pro Asp Asn Pro His Ile Cys Gly Asp Gly
65                  70                  75                  80
Thr Pro Met Glu Gly Asp Gln Phe Ser Ala Pro Leu Phe Leu Cys Thr
                85                  90                  95
Leu Pro Ala Leu Ala Ala Gly Met Leu Leu Pro Ser Arg Gly Gln Ala
            100                 105                 110
Arg His Asn Ala Thr Gly Glu Leu Asn Pro Arg Ala Cys Val Ala Thr
        115                 120                 125
Thr Cys Asp Cys Arg Gly Val Pro Pro Gly Leu Phe Cys Gly Asp Gly
    130                 135                 140
Ala Phe Gly Cys Ser Gln Gly Asp Val Phe Gln Cys Gly Val Asp Pro
145                 150                 155                 160
Gly Asn Val Cys Thr Tyr Gly Val Arg Ile Ser Cys Thr Gly Cys Thr
                165                 170                 175
Thr Val Phe Cys Glu Asp
            180

<210> 28
<211> 113
<212> PRT
<213> Auricularia

<220>
<223> gi|598049997|ref|XP_007354371.1|_Auricularia subglabra TFB -10046
      SS5

<400> 28
Met Leu Met Leu Phe Ser Leu Gln Phe Phe Thr Val Val Ala Leu Ala
1               5                   10                  15
Val Ser Ala Ala Thr Ala Val Val Ala Gln Arg Asp Pro Ala Val Leu
            20                  25                  30
Glu Ala Leu Ala Ala Arg Glu Gly Ala Glu Val Ser Ala Arg Phe Pro
        35                  40                  45
Gly Asp Gly Glu Leu Thr Lys Arg Ala Cys Ala Ala Ser Lys Cys Asn
    50                  55                  60
Cys Ser Gly Ile Lys Ala Gly Leu Trp Cys Gly Asp Gly Leu Leu Asn
65                  70                  75                  80
Cys Lys Lys Gly His Val Tyr Gln Cys Ser Thr Asp Gly His Thr Thr
                85                  90                  95
Cys Asp Tyr Gly Val Arg Thr Ser Cys Gln Lys Cys Asn Lys Leu Ala
            100                 105                 110
Cys


<210> 29
<211> 766
<212> PRT
<213> Auricularia


<220>
<223> gi|598027571|ref|XP_007343158.1|_Auricularia subglabra TFB -10046
      SS5

<400> 29
Met Ala Ser Arg Leu Ala Asp Glu Thr Leu His Gln Ile Leu Gln Ile
1               5                   10                  15
Ala Leu Glu Val Pro Asp Asp Asp Phe Ala Asp Val Ser Gly Met Lys
            20                  25                  30
Leu Ser Pro Phe Ala His Arg Glu Tyr Ser Ser Ser Asp Val Leu Leu
        35                  40                  45
Val Cys Lys Arg Trp Met Arg Val Ala Thr Pro Leu Leu Tyr Asp Thr
    50                  55                  60
Val Val Leu Arg Ser Thr Ala Gln Ala Gln Ser Leu Ala Arg Ala Leu
65                  70                  75                  80
Lys Lys Asn Pro Gln Phe Ala Pro Tyr Leu Lys Lys Leu Arg Leu Glu
                85                  90                  95
Gly Gly Phe Gly Ala Ser Val Gly Lys Ile Leu Arg Ala Thr Gly Ser
            100                 105                 110
Leu Arg Asp Leu His Leu Ser Leu Asp Leu Thr Thr Lys Asp Asn Val
        115                 120                 125
Arg Ser Leu Cys Glu Ala Leu Pro Val Ile Asn Pro Thr Arg Leu Ile
    130                 135                 140
Val Ser Ala Thr Pro Phe Ala Ser Thr Gly Ser Cys Thr Leu Ala Val
145                 150                 155                 160
Ile Asp Ala Leu Glu Asn Ala Leu Pro Ala Trp Gly Asn Thr Thr Thr
                165                 170                 175
Ala Val Val Ser Ser Leu Thr Val Leu Phe Asn Pro Gln Val Thr Met
            180                 185                 190
Leu Val Ala Lys Ile Pro Lys Ile Gln Asp Ile Trp Val Thr Lys Asn
        195                 200                 205
Arg Ser Ser Phe Leu Asp Leu Gly Leu Val Ser Ala Leu Gln Val Leu
    210                 215                 220
Pro Ala Cys Lys Ile His Val Glu Ala Asp Thr Leu Glu Thr Leu Asp

225                 230                 235                 240
Arg Thr Phe Gln Ala Ser Met Ala Ala Glu Gly Gly His Met Ile Met
                245                 250                 255
Pro Tyr Leu Lys Glu Thr Pro Thr His Glu Ile His Lys Val Ala Lys
            260                 265                 270
Asn Asp Cys Lys Ile Asn Leu Leu Arg His Val Pro Leu Glu Val Trp
        275                 280                 285
Gly Ile Val Leu Glu Tyr Val Leu Thr Thr Thr Asp Val Arg Gly Phe
    290                 295                 300
Tyr Gly Arg Leu Asp Ala Pro Leu His Cys Ser Gly Ser Leu Ser Arg
305                 310                 315                 320
Arg Leu Val Thr Thr Asn Thr Thr Ala Leu Ser Tyr Met Ser Val Cys
                325                 330                 335
Lys Leu Phe Leu Asp Val Cys Thr Gly Ile Ser Pro Arg His Met Ser
            340                 345                 350
Phe Gly Ser Asp Glu Ala Gln Ala Ser Phe Phe Arg Cys Leu Asp Arg
        355                 360                 365
Arg Pro Glu Leu Ala Phe Val Val Arg Ser Leu Ile Arg Glu Pro Ala
    370                 375                 380
Val Ala Pro Ser Gly Leu Ala Glu Cys Leu Thr Arg Leu Pro Asn Leu
385                 390                 395                 400
Thr Ser Leu Arg Cys Cys Ile Pro Leu Arg Asp Leu His Val Leu Arg
                405                 410                 415
Ala Tyr Ala Ser Arg Ala Thr Leu Val Glu Leu Glu Val Ile Ile Ala
            420                 425                 430
Pro Glu Arg Asp His Ala Pro Val Asn Phe Ser Cys Pro Ser Leu Gln
        435                 440                 445
Arg Leu Lys Ile Val His Ala Glu Gly Glu Arg Pro Gln Ser Gly Gly
    450                 455                 460
Ala Phe Gly Phe Asp Leu Pro Met Leu Gln Glu Leu Glu Leu Pro Gly
465                 470                 475                 480
Asp Asp Ala Pro Trp Thr Ile Asp Val Leu Ala Arg Thr Gln Leu Pro
                485                 490                 495
Arg Leu Arg Val Leu Arg Val Gln Ser Met Cys Arg Gln Ala Val Asp
            500                 505                 510
Glu Leu Leu Gly Thr His Gly His Ala Cys Glu Glu Val Ala Ala Arg
        515                 520                 525
Thr Arg His Leu Ala Leu Thr Leu Thr His Cys Pro Asn Met Arg Thr
    530                 535                 540
Leu Val Leu Ile Gly Val Met Glu Thr Leu Pro Leu Asp Ala Leu Ala
545                 550                 555                 560
Arg Glu Ser Pro His Ala Asn Leu Glu Thr Leu Arg Phe Thr Glu Leu
                565                 570                 575
Val Ser Gln Gly Tyr Pro Arg Lys Asp Val Lys Asn Val Ala Asp Ser
            580                 585                 590
Thr Thr Ala Phe Phe Ala Ala Leu Ser Pro Thr Met Phe Pro Ala Leu
        595                 600                 605
Arg Thr Val Val Val Glu Arg Leu Ser Trp Pro Ile Thr Pro Arg Glu
    610                 615                 620
Ile Ala Ser Asp Pro Ala Val Lys Trp Ala Glu Ser Leu Gln Ile Lys
625                 630                 635                 640
Gly Ile Gln Leu Leu Asp Lys Ser Gly Arg Ala Trp Arg Pro Arg Val
                645                 650                 655
Lys Ser Thr Gly Asp Arg Phe Phe Thr Val Val Val Leu Ala Val Ala
            660                 665                 670
Thr Ala Val Val Ala Gln Arg Asp Ala Ala Val Val Asp Ala Val Ala
        675                 680                 685
Ala Arg Glu Gly Val Glu Val Tyr Asp Ser Phe Pro Pro Gly Asp Gly
    690                 695                 700
Ala Leu Ala Lys Arg Ala Cys Val Ser Ser Ser Cys Asn Cys Ser Gly
705                 710                 715                 720
Phe Lys Pro Gly Leu Phe Cys Gly Asp Gly Leu Leu Asn Cys Lys Arg
                725                 730                 735

Gly His Val Tyr Gln Cys Ser Thr Asn Gly His Ser Thr Cys Asp Tyr
            740                 745                 750
Gly Val Arg Lys Ser Cys Gln Gln Cys Asn Lys Leu Ser Cys
        755                 760                 765

<210> 30
<211> 64
<212> PRT
<213> Auricularia

<220>
<223> gi|598039723|ref|XP_007349234.1|_Auricularia subglabra TFB -10046
      SS5

<400> 30
Asp Gly Glu Leu Val Lys Arg Ala Cys Thr Ala Ser Lys Cys Asn Cys
1               5                   10                  15
Ser Gly Ile Lys Ala Gly Leu Trp Cys Gly Asp Gly Leu Leu Asn Cys
            20                  25                  30
Lys Lys Gly His Val Tyr Gln Cys Ser Thr Asp Gly His Thr Thr Cys
        35                  40                  45
Asp Tyr Gly Val Arg Thr Ser Cys Gln Lys Cys Asn Lys Leu Ser Cys
    50                  55                  60

<210> 31
<211> 100
<212> PRT
<213> Exidia glandulosa

<220>
<223> gi|1024106614|gb|KZV97813.1|_Exidia glandulosa HHB12029

<400> 31
Met Lys Gly Ser Leu Arg Ile Val Ala Phe Thr Leu Val Leu Thr Phe
1               5                   10                  15
Cys Gln Cys Ile Leu Ala Ser Leu Leu Pro Leu Asn Val Ser His Met
            20                  25                  30
Asp Val Arg Ser Ala Leu Glu Ala Arg Asp Cys Val Ala Thr Thr Cys
        35                  40                  45
Lys Cys Thr Gly Ile Arg Thr Gly Leu Phe Cys Gly Asp Gly Ala Phe
    50                  55                  60
Asn Cys Lys Val Gly Asn Val Tyr Gln Cys Gly Glu Asn Gly Lys Ile
65                  70                  75                  80
Ser Cys Asp Tyr Gly Phe Arg Thr Ser Cys Pro Asp Cys Thr Thr Ile
                85                  90                  95
Phe Cys Glu Asn
            100

<210> 32
<211> 305
<212> PRT
<213> Exidia glandulosa

<220>
<223> gi|1024101480|gb|KZV92756.1|_Exidia glandulosa HHB12029

<400> 32
Met Pro Thr Asp Asp Asn Thr Ser Gln Thr Ala Val Ser Arg Tyr Pro

1               5                   10                  15
Lys Glu Met Pro Ala His Trp Lys Gly Glu Phe Pro Ala Ala His Leu
                20                  25                  30
Tyr Val Arg Pro Leu Leu Glu Gly Thr Trp Lys Ile Leu Cys Leu Ile
        35                  40                  45
Ala Trp Gly Thr Pro Thr Leu Glu Leu Arg Trp Ala Ser Val Arg Ala
    50                  55                  60
Glu Thr Glu Glu Glu His Thr Gln Tyr Leu Asp Gly Ile Ser Lys Arg
65                  70                  75                  80
Leu Gln Thr Thr Thr Leu Val Ala Ser Leu Leu Leu Ala Ser Asn Ala
                85                  90                  95
Ala Leu Leu Thr Thr Glu Pro Pro Leu Ser Gly Gln Ile Val Asn Tyr
            100                 105                 110
Thr Ala His Gly Pro Tyr Ile Cys Leu Trp Val Ser Phe Gly Leu Leu
        115                 120                 125
Leu Gly Ala Val Ile Val Gly Ser Gly Ile Ile Ser Leu Met Ala Thr
    130                 135                 140
Cys Thr Arg Glu Phe Ser His Asp Ala Cys Glu Met Ala Ser Asp Thr
145                 150                 155                 160
Pro Val Met Gly Asn Gly Arg Cys Ile Ala Leu Lys Ile Thr Pro Asp
                165                 170                 175
Glu Glu Asp Arg Gly Leu Val Gly Arg Trp Ser Ala Gly Val Ala Gly
            180                 185                 190
Arg Thr Ile Ala Thr Leu Ser Pro Leu Ala Ile Asp Asp Asp Met Lys
        195                 200                 205
Gly Ser Leu Arg Asn Val Ala Phe Thr Leu Val Val Thr Ile Cys Gln
    210                 215                 220
Tyr Val Thr Ala Ser Leu Leu Pro Pro Asn Val Ser His Leu Asp Val
225                 230                 235                 240
Arg Ser Ala Leu Glu Ala Arg Asp Cys Val Ala Thr Thr Cys Gln Cys
                245                 250                 255
Thr Gly Ile Arg Thr Gly Leu Phe Cys Gly Asp Gly Ala Phe Asn Cys
            260                 265                 270
Lys Val Gly Asn Val Tyr Gln Cys Gly Glu Asn Gly Gln Ile Ser Cys
        275                 280                 285
Asp Tyr Gly Phe Arg Glu Ser Cys Pro Asp Cys Thr Val Val Phe Cys
    290                 295                 300
Glu
305

<210> 33
<211> 709
<212> PRT
<213> Exidia glandulosa


<220>
<223> gi|1024104254|gb|KZV95484.1|_Exidia glandulosa HHB12029

<400> 33
Met Phe Pro Arg Val Leu Ala Val Ala Val Leu Leu Phe Val Ser Leu
1               5                   10                  15
Thr Val Ala Gln Ser Asn Ala Thr Glu Leu Ile Asn Gln Ile Ala Ala
            20                  25                  30
Asp Ile Glu Ser Ala Val Asp Cys Ala Ser Cys His Ala Leu Leu Ile
        35                  40                  45
Pro Leu Lys Ala Leu Ala Leu Leu Gly Asp Asp Val Phe Thr Gly Gly
    50                  55                  60
Ile Val Gly Ile Cys Asp Leu Ser Gly Ala Glu Asp Pro Asp Val Cys
65                  70                  75                  80
Ala Gly Ala Val Gly Gly Gln Ala Pro Ile Ile Ala His Gly Leu Arg
                85                  90                  95
Leu Ile Asp Pro Leu Gly Gln Thr Ala Thr Lys Leu Cys Asn Ala Leu

            100                 105                 110
Phe Gly Leu Cys Ala Leu Pro Pro Val Asn Glu Phe Thr Leu Pro Leu
        115                 120                 125
Pro Pro Ala Pro Ala Ser Pro Lys Lys Phe Val Ser Arg Gly Arg Pro
    130                 135                 140
Pro Leu Thr Val Ala His Leu Ser Asp Val His Ile Asp Arg Glu Tyr
145                 150                 155                 160
Thr Val Gly Ala Asp Ala Gln Cys Thr Lys Pro Leu Cys Cys Arg Asn
                165                 170                 175
Phe Ala Asp Gln Ala Gly Gln Pro Ile Ala Glu Pro Ala Gly Pro Ser
            180                 185                 190
Gly Asn Ser Asn Cys Asp Ser Pro Val Lys Leu Ala Asp Ala Met Leu
        195                 200                 205
Ser Ala Val Lys Gly Leu Asn Pro Asp Phe Ala Ile Phe Thr Gly Asp
    210                 215                 220
Ile Val Glu Gly Ala Ile Trp Leu Val Asp Arg Ala Glu Val Thr Met
225                 230                 235                 240
Asp Leu Ser Ala Phe Asp Asp Glu Met Gly Ser Leu Leu Gly Thr Ile
                245                 250                 255
Pro Val Phe Pro Ala Ile Gly Asn His Asp Ser Ala Pro Val Asn Ser
            260                 265                 270
Phe Pro Arg Asp Glu Ala Ala Leu Thr Ala Ala Asn Asn Ser Ala Ser
        275                 280                 285
Ser Ser Gln Trp Val Phe Asp Thr Gln Ala Gln Gly Trp Gln Arg Trp
    290                 295                 300
Ile Asn Thr Thr Ala Ala Gln Glu Val Arg His Ile Ser Gly Ser Tyr
305                 310                 315                 320
Gly Ala Val Val Pro Gly Thr Lys Leu Arg Ile Ile Ser Leu Asn Thr
                325                 330                 335
Gln Tyr Tyr Tyr Lys Gln Asn Phe Trp Leu Tyr Asp Thr Asp Ala Val
            340                 345                 350
Val His Asp Pro Gln Gly Leu Leu Ala Phe Val Gly Ser Gln Leu Gln
        355                 360                 365
Leu Ala Glu Thr Leu Gly Glu Arg Val Trp Ile Ile Gly His Ile Ser
    370                 375                 380
Ser Gly Lys Thr Asp Phe Asn Arg Asp Pro Ser Asn Tyr Phe Asn Gln
385                 390                 395                 400
Leu Val Gln Arg Tyr Ser Asn Thr Ile Ala Gly Asn Phe Phe Gly His
                405                 410                 415
Ser His Lys Asp Gln Phe Glu Ile Thr Tyr Lys Asp Phe Asn Asp Arg
            420                 425                 430
Thr Ala Ala Asn Ala Asn Ser Val Asn Ile Ile Ser Pro Ala Leu Thr
        435                 440                 445
Pro Thr Ser Gly Asn Pro Ala Phe Lys Phe Tyr Asp Ile Asp Pro Asp
    450                 455                 460
Thr Tyr Glu Leu Met Asp Val Arg Val Phe Phe Ala Asn Val Ser Ser
465                 470                 475                 480
Pro Thr Phe Gln Thr Ser Pro Thr Phe Glu Leu Tyr Tyr Ser Ala Arg
                485                 490                 495
Glu Ser Tyr Gly Ala Leu Leu Pro Gln Pro Ile Pro Ala Thr Gln Pro
            500                 505                 510
Leu Ser Pro Ala Phe Trp His Asn Leu Thr Glu Val Phe Glu Ser Asp
        515                 520                 525
Asp Glu Ala Phe Gln Leu Tyr Asn Glu Arg Leu Ser Arg Gly Gly Ala
    530                 535                 540
Val Gly Ala Cys Asp Ala Asp Cys Lys Ala Asn Thr Ile Cys Asn Leu
545                 550                 555                 560
Arg Ala Ala Arg Ser Glu Asn Asn Cys Asp Val Ala Thr Pro Gly Leu
                565                 570                 575
Ser Phe Lys Arg Ser Leu Pro Arg His Ser Glu Glu Ser Glu Cys Glu
            580                 585                 590
Gly Leu Ser Ile Ala Thr Val Phe Arg Ser Ala Phe Ile Ser Ile Ile
        595                 600                 605

Ala Leu Ala Val Ser Ala Ala Thr Ala Val Val Ala Gln Arg Asp Pro
    610                 615                 620
Ala Val Leu Ala Ala Leu Asn Ala Arg Pro Gly Phe Glu Ala Arg Glu
625                 630                 635                 640
Ile Ser Asp Phe Glu Gly Gly Ser Leu Val Lys Arg Ala Cys Thr Ala
                645                 650                 655
Ser Ser Cys Asn Cys Ser Gly Phe Pro Pro Gly Leu Phe Cys Gly Asp
            660                 665                 670
Gly Val Leu Asn Cys Lys Lys Gly Asn Val Tyr Gln Cys Ser Thr Asp
        675                 680                 685
Gly His Thr Thr Cys Ser Tyr Gly Val Arg Thr Ser Cys Gln Lys Cys
    690                 695                 700
Asn Ala Leu Thr Cys
705

<210> 34
<211> 84
<212> PRT
<213> Artificial Sequence

<220>
<223> jgi|Umbra1|249846|_combest_scaffold_44_94105

<400> 34
Met Leu Ser Ser Met Lys Leu Leu Val Leu Ala Leu Cys Val Phe Ser
1               5                   10                  15
Leu Met Ser Ser Val Ser Cys Asn Leu Glu Lys Arg Gln Cys Ser Ile
            20                  25                  30
Ser Ser Cys Ser Cys Gln Gly Val Val Asp Gly Leu Phe Cys Gly Asp
        35                  40                  45
Gly Arg Tyr Asn Cys Lys Glu Gly Arg Leu Tyr Glu Cys Gln Asn Gly
    50                  55                  60
Gly Arg Ala Cys Asp Tyr Gly Val His Asp Ser Cys Ala Lys Cys Asn
65                  70                  75                  80
His Leu Thr Cys

<210> 35
<211> 98
<212> PRT
<213> Artificial Sequence

<220>
<223> jgi|Mictr1|637882|gm1.4452_g

<400> 35
Met Pro Phe Trp Asn Met Gly Lys Met Gln Phe Asp Phe His Ser Arg
1               5                   10                  15
Leu Glu Tyr Ser Val Ile Leu Ser Ile Ala Ala Ala Ala Leu Pro Leu
            20                  25                  30
Val Leu Gly Gly Glu Val Thr Pro Arg Asp Cys Ala Pro Thr Lys Cys
        35                  40                  45
Ser Cys His Ala Ile Pro Asp Gly Leu Phe Cys Gly Asp Gly Leu Leu
    50                  55                  60
Asp Cys Thr Pro Gly His Val Tyr Gln Cys Gly Gly Asp Gly Lys Lys
65                  70                  75                  80
Ser Cys Asp Phe Gly Pro Arg Asp Ser Cys Gln Lys Cys Gly Glu Leu
                85                  90                  95
Glu Cys

<210> 36
<211> 57
<212> PRT
<213> Artificial Sequence

<220>
<223> Piriformospora_indica_PIIN_03211, conserved 1

<400> 36
Ala Cys Asn Tyr Asn Thr Pro Cys Lys Gly Tyr Tyr Trp Ser Lys Gly
1               5                   10                  15
Leu His Cys Gly Asp Gly Asn Tyr Gly Cys Val Tyr Gly His Val Tyr
            20                  25                  30
Gln Val Gly Ser Asp Thr Asn Val Cys Asp Tyr Gly Val Arg Thr Ser
        35                  40                  45
Cys Gln Lys Cys Gly Lys Leu Ser Cys
    50                  55

<210> 37
<211> 40
<212> PRT
<213> Artificial Sequence

<220>
<223> Piriformospora_indica_PIIN_03211, conserved 2

<400> 37
Gly Leu His Cys Gly Asp Gly Asn Tyr Gly Cys Val Tyr Gly His Val
1               5                   10                  15
Tyr Gln Val Gly Ser Asp Thr Asn Val Cys Asp Tyr Gly Val Arg Thr
            20                  25                  30
Ser Cys Gln Lys Cys Gly Lys Leu
        35                  40

<210> 38
<211> 126
<212> PRT
<213> Serendipita indica

<220>
<223> G4TDB9

<400> 38
Met Pro Asp Thr Ala Ala Val Ile Lys Gln Val Gln Thr Glu Ala Gln
1               5                   10                  15
Glu Arg Ile Lys Glu Ala Leu Gln Phe Thr Thr Val Phe Val Thr Leu
            20                  25                  30
Ala Ile Phe Ala Ser Ser Ala Phe Ala Ala Ala Val Ala Asp Asp Glu
        35                  40                  45
Thr Ala Val Ile Ala Arg Ala Arg Ser Glu Asp Ala Ala Gly Ser Val
    50                  55                  60
Asp Leu Leu Lys Arg Ala Cys Asn Tyr Asn Thr Pro Cys Lys Gly Tyr
65                  70                  75                  80
Tyr Trp Ser Lys Gly Leu His Cys Gly Asp Gly Asn Tyr Gly Cys Val
                85                  90                  95
Tyr Gly His Val Tyr Gln Val Gly Ser Asp Thr Asn Val Cys Asp Tyr
            100                 105                 110
Gly Val Arg Thr Ser Cys Gln Lys Cys Gly Lys Leu Ser Cys
        115                 120                 125

<210> 39
<211> 381
<212> DNA
<213> Serendipita indica

<220>
<223> CCA69312

<400> 39
atgcccgata ctgcagccgt catcaaacaa gtccaaacag aggcccagga gcgtataaaa 60

gaggctttac agttcactac cgtcttcgtc accctcgcca tcttcgcctc ctctgctttc 120

gcagcagctg tcgccgatga cgaaacagcg gtcattgctc gtgctcgcag cgaggatgct 180

gctggaagtg tcgatcttct caagcgcgct tgcaactata acacgccttg caagggatac 240

tactggtcca aagggctcca ctgtggcgat ggcaactatg gatgcgtcta cggccacgtc 300

taccaggttg gttctgacac caacgtctgt gactatggtg ttcggacctc ttgccagaag 360

tgcgggaagc tctcgtgcta g 381

<210> 40
<211> 11
<212> PRT
<213> Artificial Sequence

<220>
<223> Motif 1 of SEQ ID NO: 2

<400> 40
Cys Asp Tyr Gly Val Arg Thr Ser Cys Gln Lys
1               5                   10

<210> 41
<211> 5
<212> PRT
<213> Artificial Sequence

<220>
<223> Motif 2 of SEQ ID NO: 2

<400> 41
Gly His Val Tyr Gln
1               5

<210> 42
<211> 7
<212> PRT
<213> Artificial Sequence

<220>
<223> Motif 3 of SEQ ID NO: 2

<400> 42
Gly Leu His Cys Gly Asp Gly
1               5

<210> 43
<211> 23
<212> PRT
<213> Artificial Sequence

<220>
<223> Motif 4 of SEQ ID NO: 2

<400> 43
Gly His Val Tyr Gln Val Gly Ser Asp Thr Asn Val Cys Asp Tyr Gly
1               5                   10                  15
Val Arg Thr Ser Cys Gln Lys
            20